Europäisches Patentamt

European Patent Office

Office européen des brevets

(11) Publication number: **0 265 156**
**A2**

(12) # EUROPEAN PATENT APPLICATION

(21) Application number: 87309057.5

(22) Date of filing: 14.10.87

(51) Int. Cl.⁴: **C12P 21/00** , C12N 15/00 ,
C12N 5/00 , G01N 33/577 ,
G01N 33/76 , A61K 37/24 ,
A61K 39/395 , A61K 49/00

The microorganisms have been deposited with the European Collection of Animal Cell Cultures (ECACC) under numbers 87100203 and 87100204.

A request for correction of page 43 of the originally filed description has been filed pursuant to Rule 88 EPC. A decision on the request will be taken during the proceedings before the Examining Division (Guidelines for Examination in the EPO, A-V, 2.2).

(30) Priority: **14.10.86 AU 8482/86**
**16.12.86 AU 9525/86**

(43) Date of publication of application:
**27.04.88 Bulletin 88/17**

(84) Designated Contracting States:
**AT BE CH DE ES FR GB GR IT LI LU NL SE**

(71) Applicant: **Bunge (Australia) Proprietary Limited**
**6th Floor 616 St. Kilda Road**
**Melbourne Victoria 3004(AU)**

(72) Inventor: **Mountford, Peter Scott**
**45 Waterloo Crescent**
**St. Kilda Victoria(AU)**

(74) Representative: **Harding, Richard Patrick et al**
**Arthur R. Davies & Co. 27 Imperial Square**
**Cheltenham GL50 1RQ(GB)**

(54) **Monoclonal antibodies.**

(57) A monoclonal antibody against equine chorionic gonadotrophin produced from a continuous cell line which produces a monoclonal antibody against equine chorionic gonadogrophin, including a hybridoma formed by fusina a B cell capable of producing antibodies against equine chorionic gonadotrophin with a myeloma cell and its use in purifying equine chorionic gonadotrophin and modifying the biological activity of equine chorionic gonadotrophin.

EP 0 265 156 A2

The present invention relates to monoclonal antibodies, a method for the preparation thereof and use thereof.

Equine chorionic gonadotrophin or pregnant mare serum gonadotrophin was first described by Cole and Hart [Anat. Rec., 56 , 275-293 (1930)] as anterior hypophyseal sex maturing hormone due to its potent stimulatory effect on the sexual maturity of the immature rat. Its presence was noted in the sera of pregnant mares from approximately day 37 till day 100 of pregnancy.

More recently PMSG has been referred to as equine chorionic gonadotrophin (eCG), a name chosen to reflect the source of the hormone and its close homology with human chorionic gonadotrophin (hCG).

eCG has a potent, dual LH-FSH bioactivity capable of inducing follicular growth and ovulation when injected into a wide range of domestic animals. The hormone has a particularly long half life, 21.2 hours in sheep, 50-120 hours in cattle and 26 hours in rats which is in part responsible for the high biological potency of the hormone in these species. This potent dual LH-FSH bioactivity of eCG in non-equine species has led to descriptions of the hormone such as "very nearly the complete gonadotrophin". Single administrations of the hormone are capable of inducing follicular growth, oestrogen production, ovulation, luteinization and subsequent progesterone synthesis in sheep, cattle, goats, pigs, dogs, cats, rats and mice.

eCG is extensively used in veterinary practice for superovulation in embryo transfer programs, induction of ovulation and oestrus in anoestrus animals and for mild ovulation increases to give greater fecundity. However, variation in the response of treated animals is a major problem and may be attributed to batch variation and contaminants present in the commercial eCG preparations that are currently available. Seasonal effects including follicular/ovarian status of the treated animals also has an effect. A further problem exists with superovulatory doses where the contaminating substances and the higher dose of eCG causes continued stimulation of the ovaries and the production of persistent secondary follicles. These follicles often become large and cystic, causing excessive oestrogen production and eventually contractile scar tissue in the ovaries. Excessive oestrogen can have deleterious effects on egg, sperm and embryo transfer within the reproductive tract of rabbits, rats, mice, guinea pigs, and domestic farm animals.

Particular difficulties are further encountered with respect to the prior art relating to methods of production and purification of eCG.

Two major extraction methods are used for the production of crude extracts of eCG from pregnant mare sera; (i) acid and ethanol precipitation, and (ii) acetone precipitation. Depending on the method used, unwanted serum proteins are precipitated by the addition of ethanol or acetone to 50% (v/v). The further addition of ethanol or acetone to 70-75% then precipitates the active component. The technique of Gospodarowicz and Papkoff, [Endocrinology, 80, 699-702 (1967)] produces crude eCG extracts of approximately 600iu/mg of protein while the Cartland and Nelson [J.Biol. Chem. 119 , 59-67(1937)] method results in a crude extract containing 50-100iu/mg of protein.

Further purification steps using the crude serum extracts include gel filtration and sulphoethyl-Sephadex ion exchange chromatography (Gospodarowicz and Papkoff, 1967), and gel filtration and hydroxylapatite chromatography Moore and Ward, [J.Biol. Chem., 255 , 6923-6929, 1980]. Others have used wheat germ lectin Sepharose chromatography. The purified products obtained range in potency from 9,000-15,800iu/mg, however estimations of specific activity are often unreliable as the measurements are based on standards of variable quality. Commercial preparations range from 325 iu/mg to 2500 iu/mg or approximately 5-20% purity.

Accordingly, it is an object of the present invention to overcome, or at least alleviate, one or more of the difficulties related to the prior art.

Accordingly, in a first aspect, the present invention provides a process for producing a monoclonal antibody against equine chorionic gonadotrophin (eCG) which method includes

(a) providing a B cell capable of producing antibodies against equine chorionic gonadotrophin, and a myeloma cell,

(b) fusing the B cell with the myeloma cell, and

(c) propagating a hybridoma formed thereby.

The process for producing a monoclonal antibody according to this aspect of the present invention may further include

(d) harvesting an antibody produced by said hybridoma.

The B cell capable of producing antibodies against eCG may be selected from spleen cells and lymph node cells of a test animal. The test animal may be a mouse, rat or the like. The B cells may be obtained from an animal immunised with eCG or an immunogenic fragment thereof. A BALB/c mouse is the preferred animal for immunisation. Once-primed or hyper-immunised animals may be used as the source of antibody producing lymphocytes or splenocytes. Mouse and rat splenocytes give a higher percentage of stable fusions with a mouse myeloma line described below. However, the use of rat, human and other mammalian cells as well as frog cells is also contemplated. In a particularly preferred form, hyperimmunised mouse spleen cells are used to make the fused cell hybrids.

The myeloma cell may be of any suitable type. Specialized myeloma cell lines which have been developed from lymphocyte tumours for use in hybridoma-producing fusion procedures are known in the prior art. The cell lines have been developed for at least three reasons. The first is to facilitate the selection of fused hybridomas among unfused and similarly indefinitely self-propagating myeloma cells. Usually, this is accomplished by using myelomas with enzyme deficiencies that render them incapable of growing in certain selective media that support the growth of hybridomas. The second reason arises from the inherent ability of lymphocyte tumour cells to produce their own antibodies. The purpose of using monoclonal techniques is to obtain immortal fused hybrid cell lines that produce the desired single specific antibody genetically directed by the somatic cell component of the hybridoma. To eliminate the production of tumour cell antibodies by the hybridomas, myeloma cell lines incapable of producing light or heavy immunoglobulin chains or those efficient in antibody secretion mechanisms are used. Third reason for selection of cell lines is for their suitability and efficiency for fusion.

Several myeloma cell lines may be used for the production of fused cell hybrids, including P3/X63-Ag 8, NS-I type myeloma cells, e.g. P3/NSI/1-Ag 4-1, Sp2/0-Ag14 and S194.5.XXO.BU.1. The P3/X63-Ag 8 and P3/NSI/1-Ag 4-1 cells lines have been described by Kohler and Milstein [Europ. J. Immunol. 6: 511-519 (1976)]. Shulman et al. [Nature 276: 269-270 (1978)] developed the Sp2/0-Ag14 myeloma line. The S194/5.XXO.BU.1 myeloma line was reported in an article by Trowbridge [J. Exp. Med. 148: 313 (1979)]. In the example of the present invention, a NS-1-type myeloma cell (derived from BALB/c mice) is the preferred cell line.

The fusion step according to this aspect of the present invention for generating hybrids of antibody-producing spleen or lymph node cells and myeloma cells includes mixing somatic cells with myeloma cells. The proportion may vary from approximately 20:1 to 1:1, preferably 10:1, respectively. The fusion step may be carried out in the presence of an agent or agents that promote the fusion of cell membranes. It is preferred that the same species of animal serve as the source of the somatic and myeloma cells used in the fusion procedure. The fusion-promoting agents used may include Sendai virus or polyethylene glycol (PEG).

Because fusion procedures produce viable hybrids at very low frequency (e.g., when spleens are used as a source of somatic cells, only one hybrid is obtained for roughly every $2 \times 10^5$ spleen cells), it is preferred to have a means of selecting the fused cell hybrids from the remaining unfused cells, particularly the unfused myeloma cells. A means of detecting the desired antibody-producing hydridomas among other resulting fused cell hybrids is also necessary.

Generally, the selection of fused cell hybrids is accomplished by culturing the cells in media that support the growth of hydridomas but prevent the growth of the myeloma cells which normally would go on dividing indefinitely. These cells are selected against in hypoxanthine/aminopterin/thymidine (HAT) medium, a medium in which the fused cell hybrids survive due to the HPRT-positive genotype of the spleen cells. The use of myeloma cells with different genetic deficiencies (e.g., other enzyme deficiencies, drug sensitivities, etc.) that can be selected against in media supporting the growth of genotypically competent hybrids is also possible.

Several weeks may be required to selectively culture the fused cell hybrids. Early in this time period, it is preferred to identify those hybrids which produce the desired antibody so that they may be subsequently cloned and propagated. Generally, around 10% of the hybrids obtained produce the desired antibody, although a range of from 1 to 30% is not uncommon.

The use of highly discriminatory screening assays during the cloning and selection of hybridoma cell lines assists in selecting for monoclonal antibodies having the desired specificity.

The detection of antibody-producing hybrids can be achieved by any one of several standard assay methods, including enzyme-linked immunoassay and radioimmunoassay techniques. Immunohistology can also be used for antibody detection and selection. The detection method preferred is an enzyme-linked immunoassay employing a perioxidase-conjugated anti-mouse immunoglobulin.

Once the desired fused cell hydrids have been selected and cloned into individual antibody-producing cell lines, each cell line may be propagated in either of two standard ways. A sample of the hybridoma can be injected into a histocompatible animal of the type that was used to provide the somatic and myeloma cells for the original fusion. The injected animal develops tumours secreting the specific monoclonal antibody produced by the fused cell hybrid. The body fluids of the animal, such as serum or ascites fluid, can be tapped to provide monoclonal antibodies in high concentration. Alternatively, the individual cell lines may be propagated in vitro in laboratory culture vessels; the culture medium, also containing high concentrations of a single specific monoclonal antibody, can be harvested by decantation, filtration or centrifugation.

Accordingly, in a further aspect of the present invention there is provided continuous cell lines which produce monoclonal antibodies against equine chorionic gonadotrophin including a hybridoma formed by fusing a B cell capable of producing antibodies against equine chorionic gonadotrophin with a myeloma cell.

Preferably, the hybridoma cell line is formed by fusing an NS-1 myeloma cell with a spleen B cell obtained from a BALB/c mouse immunised with equine chorionic gonadotrophin.

Hybridomas of interest may be dilution cloned to ensure the selection of a single cell line. Hybridomas may be dilution cloned at least four times. Particular examples of hybridoma cell lines are those designated F50-37, F50-75 and F51-99 as hereinafter described samples of which are maintained in the Bunge (Australia) Pty. Ltd. Cell Collection at 89 Flemington Road, North Melbourne, Australia. The invention described herein is not limited in scope by the cell lines exemplified since these embodiments are intended as illustrations of one aspect of the invention and any equivalent cell lines which produce a functionally equivalent monoclonal antibody are within the scope of the invention.

The hybridomas described above are a result of a fusion between a mouse splenocyte and a myeloma cell line classified as NS-1. The lymphocyte was from a spleen obtained from an inbred mouse representative of the BALB/c strain. The mouse had been immunised with eCG of high purity combined with an adjuvant. The hybridoma cell line will grow in tissue culture using a nutrient solution. The cell line when analysed for purity after repeated tissue culture passage by dilution cloning will exhibit antibody producing eCG specific clones at a frequency of 100%.

In a still further aspect of the present invention there is provided monoclonal antibodies against equine chorionic gonadotrophin.

The monoclonal antibodies may be produced from a hybridoma cell line as described abvoe. Specific examples of monoclonal antibodies include those formed by hybridoma cell lines F50-37, F50-75 and F51-99, samples of which are maintained in the Cell Collection facility of Bunge (Australia) Pty. Ltd. at 89 Flemington Road, North Melbourne, Australia.

Samples of the following hybridoma cell lines have been deposited with the European Collection of Animal Cell Cultures (ECACC).

| Culture | Deposit No. |
|---------|-------------|
| F50-37 | 87100203 |
| F51-99 | 87100294 |

## Some Physicochemical and Biological Properties of three specific Monoclonal Antibodies to eCG and the Cell Lines which produce them

### (i) Radioimmunoprecipitation of eCG

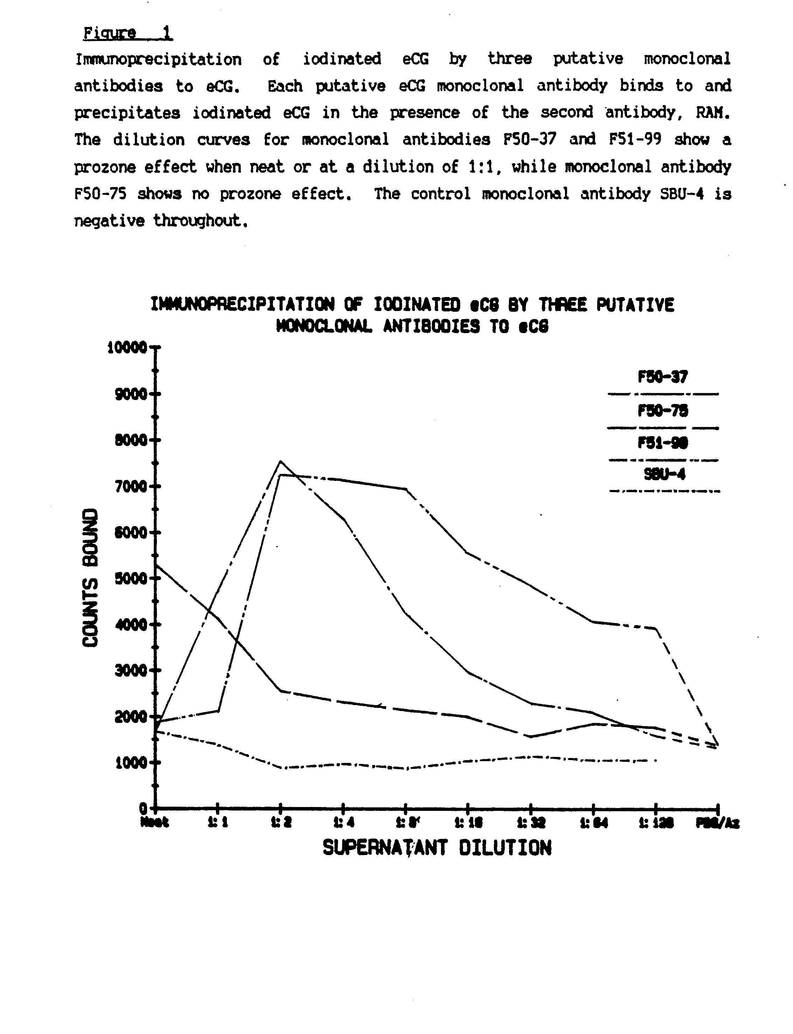

The three putative monoclonal antibodies to eCG gave positive binding reactions with $^{125}$I eCG (Figure 1). Monoclonal antibodies F50-37 and F51-99 showed considerably stronger binding than F50-75 at lower dilutions. Monoclonal antibody F50-37 gave a classic antibody dilution curve tapering to background levels in eight doubling dilutions, while F51-99 gave a dilution curve which failed to return to baseline levels over the dilution range. Both monoclonal antibodies F50-37 and F51-99 had similar maximum binding capacities at a dilution of 1:4, and no detectable binding when neat. This prozone effect when neat was notably absent with monoclonal antibody F50-75.

### (ii) Isotyping

All three monoclonal antibodies are of the IgG class, having kappa light chains. Monoclonal antibody F50-37 is IgG$_{2a}$ while F50-75 and F51-99 are both IgG$_1$. Media containing no mouse immunoglobulin and normal mouse serum gave reliable results in the assay used.

### (iii) Scatchard plot analysis

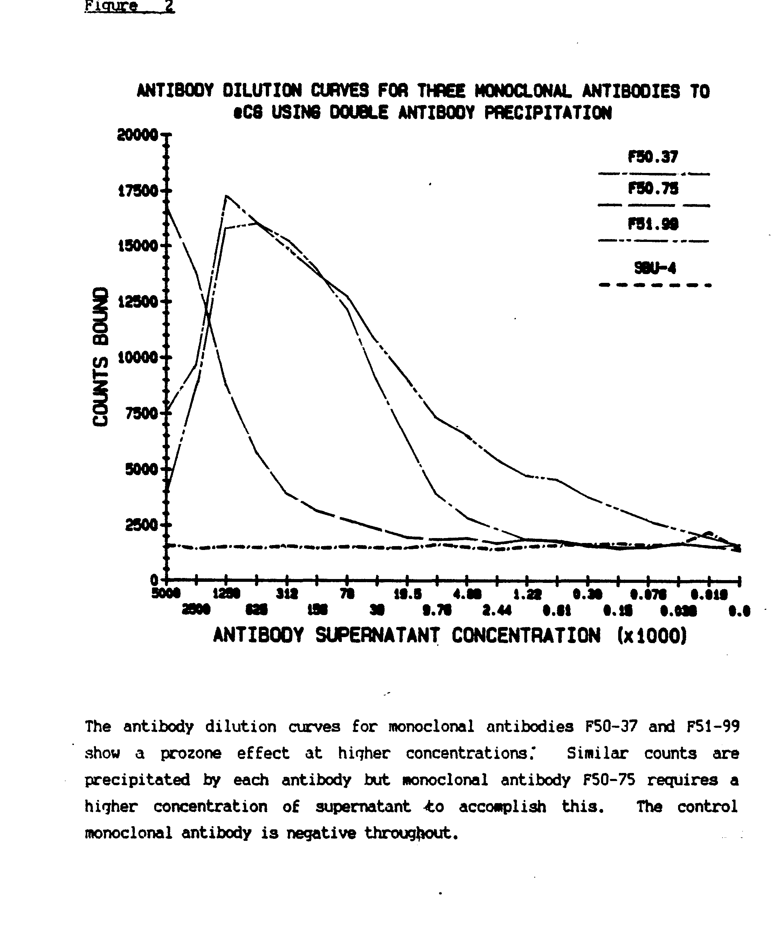

The antibody dilution curves showed monoclonal antibodies F50-37 and F51-99 to be more potent binders of $^{125}$I eCG than monoclonal antibody F50-75 at lower dilutions (Figure 2). Monoclonal antibodies F50-37 and F51-99 also had similar maximal capacities and showed a prozone effect similar to that seen in the earlier radio-immunoprecipitation experiment. The prozone effect was due to saturation of the second antibody. The antibody dilution curve for monoclonal antibody F51-99 over the expanded dilution range returned to background levels. Monoclonal antibody F50-75 showed no prozone effect and required higher concentrations of supernatant to bind the equivalent maximal counts of the other two supernatants. The control supernatant was negative throughout.

By convention, the selection of the antibody concentration from antibody dilution curves for use in the development of standard curves is that concentration capable of binding 50% of maximal counts, or, the concentration associated with the steepest part of the curve. These two criteria may coincide and are selected to give range and maximal sensitivity to the assay developed.

The following monoclonal antibody supernatant concentrations were chosen from the antibody dilution curves for standard curve construction.

F50-37     0.078 x conc.
F50-75     2.185 x conc.
F51-99     0.078 x conc.

These supernatant concentrations gave similar maximum binding capacities and reflected suitably steep regions of each antibody dilution curve.

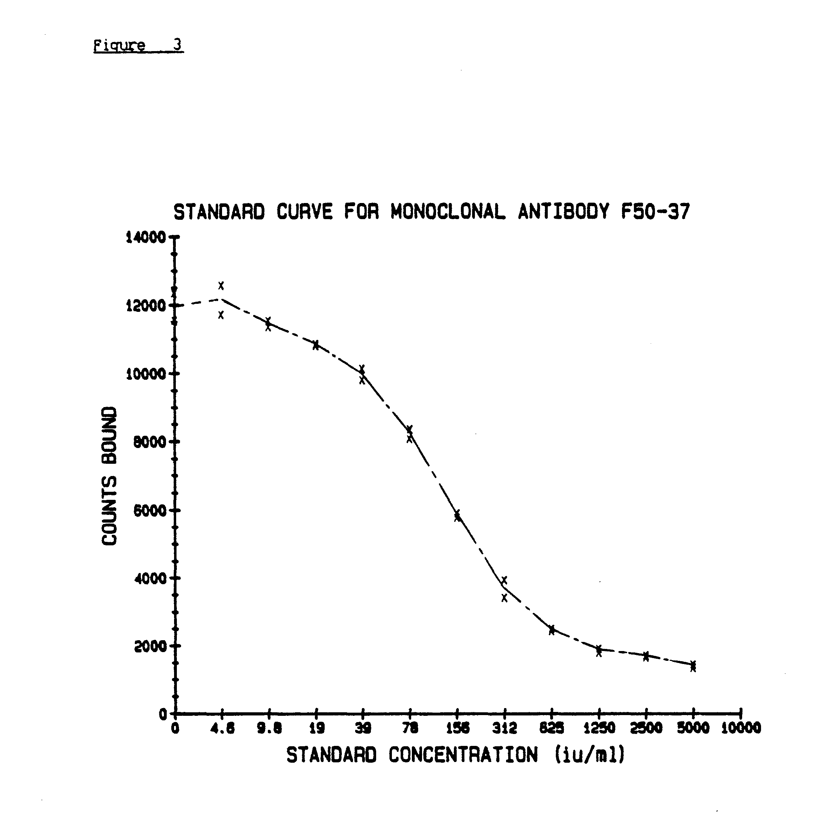

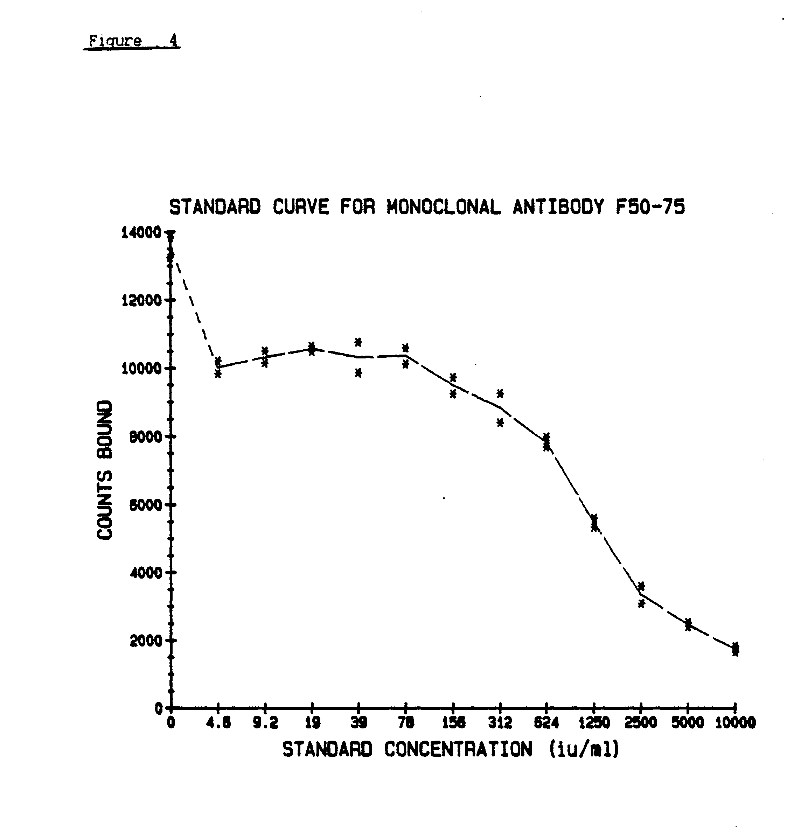

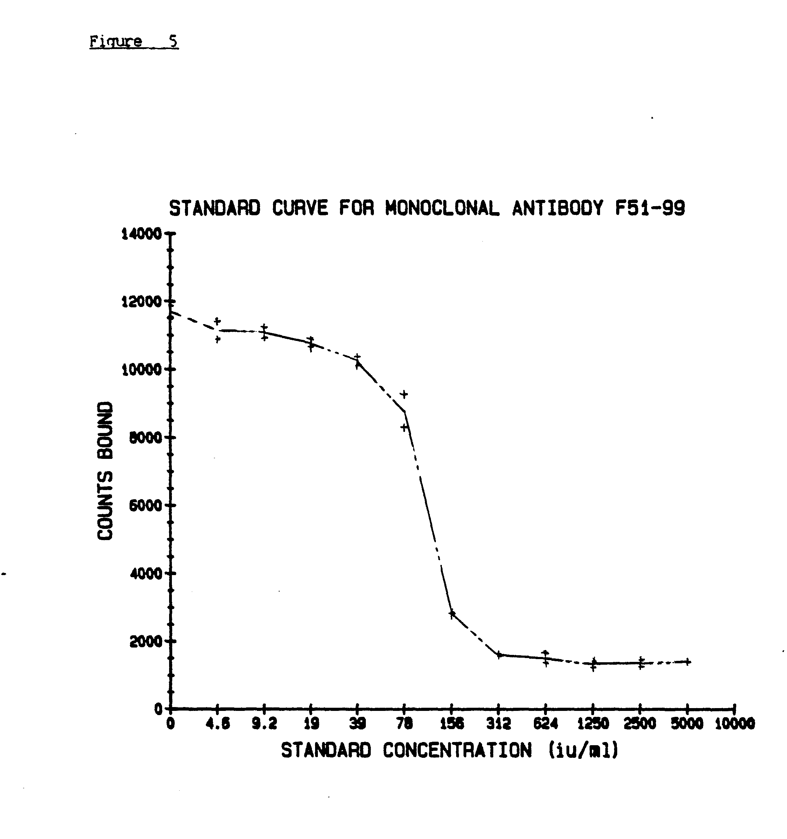

Examination of the standard curves for the three eCG monoclonal antibodies (Figures 3, 4 and 5) reveal obvious differences in shape and position. Each point shown on the standard curves is the mean of a duplicate set of tubes for one experiment and the line plotted between them is the mean result of 2 similar experiments.

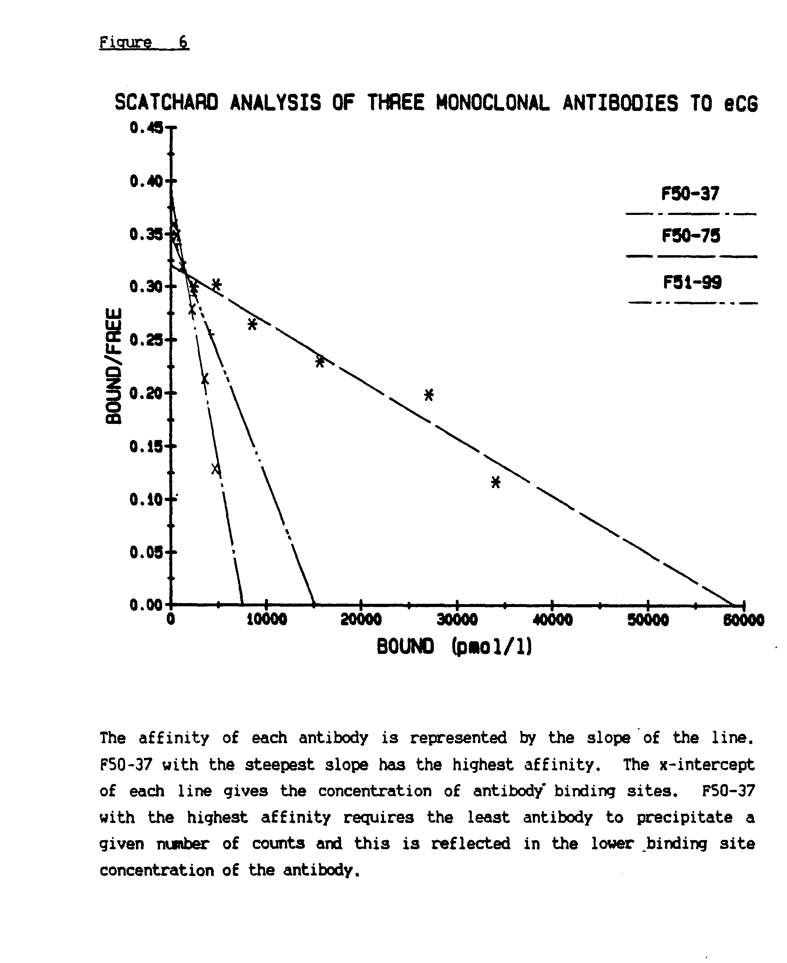

For affinity comparison, this data is presented as a Scatchard plot (Figure 6). The slope of each line is the measure of its affinity. Monoclonal antibody F50-37 has the highest affinity, while F50-75 has the lowest affinity. Computer analysis of the data found the affinities for each monoclonal antibody to be,

F50-37     $1.92 \times 10^{-8}$ mol/l
F50-75     $1.84 \times 10^{-7}$ mol/l
F51-99     $4.27 \times 10^{-8}$ mol/l

The x intercept of the line in the Scatchard plot gives the binder concentration, or, the concentration of antibody binding sites. These are by computer analysis,

F50-37     7.5 nmol/l
F50-75     52.0 nmol/l
F51-99     14.9 nmol/l

As antibody supernatant concentrations were selected to give similar $^{125}$I eCG maximal binding capacities for all three antibodies, it would be expected that higher affinity antibodies would require less total antibody to bind a given number of $^{125}$I eCG counts when the reactions are at equilibrium. That is, affinity and antibody binding site concentration should be inversely related for each antibody. Comparing affinities and binding site concentrations for the three monoclonal antibodies shows this to be true. Monoclonal antibody F50-37 with the highest affinity has the lowest concentration of binding sites and monoclonal antibody F50-75 with the lowest affinity has the highest concentration of binding sites.

By extrapolatin of binding site concentrations determined through Scatchard analysis, supernatant antibody concentrations can be determined.

Monoclonal antibody F50-37 diluted to 0.078x and having 7.49 nmol/l at this dilution would therefore have an original supernatant binding site, or antibody concentration, of

$$\frac{7.49}{0.078} \text{ nmol/l}$$

$$\text{or} \qquad 14.4 \text{ ug/ml}$$

By Scatchard analysis and extrapolation of the results, supernatant antibody concentrations of 3.6ug/ml and 28.7ug/ml were obtained for monoclonal antibodies F50-75 and F51-99 respectively.

### (iv) Agglutination of eCG coated sheep red blood cells

The three eCG monoclonal antibody supernatants, and one negative control supernatant, were titrated in 96 well round bottom trays (Flow Labs, USA) by doubling dilution of 50ul volumes from neat to 1:16 in PBS/Az. 10ul of eCG coated sheep red blood cells (SRBC) as supplied in The MIP Test Kit (Carter and Wallace, USA) were added to each well and thoroughly mixed prior to settling. After 2-3 hours settling at room temperature, the agglutinating ability of each monoclonal antibody was assessed according to cell settling patterns. An even mat of cells represented strong agglutination, whereas a discrete dark point of cells represented a negative reaction or no agglutination.

Supernatant of the monoclonal antibody F50-75 demonstrated considerably more agglutinating ability from neat to a 1:4 dilution than did supernatants of the monoclonal antibodies F50-37 and F51-99 which both showed only weak agglutination over the same dilution range. The negative control supernatant showed no agglutination.

### (v) Immunohistological characterization of three eCG monoclonal antibody specificities

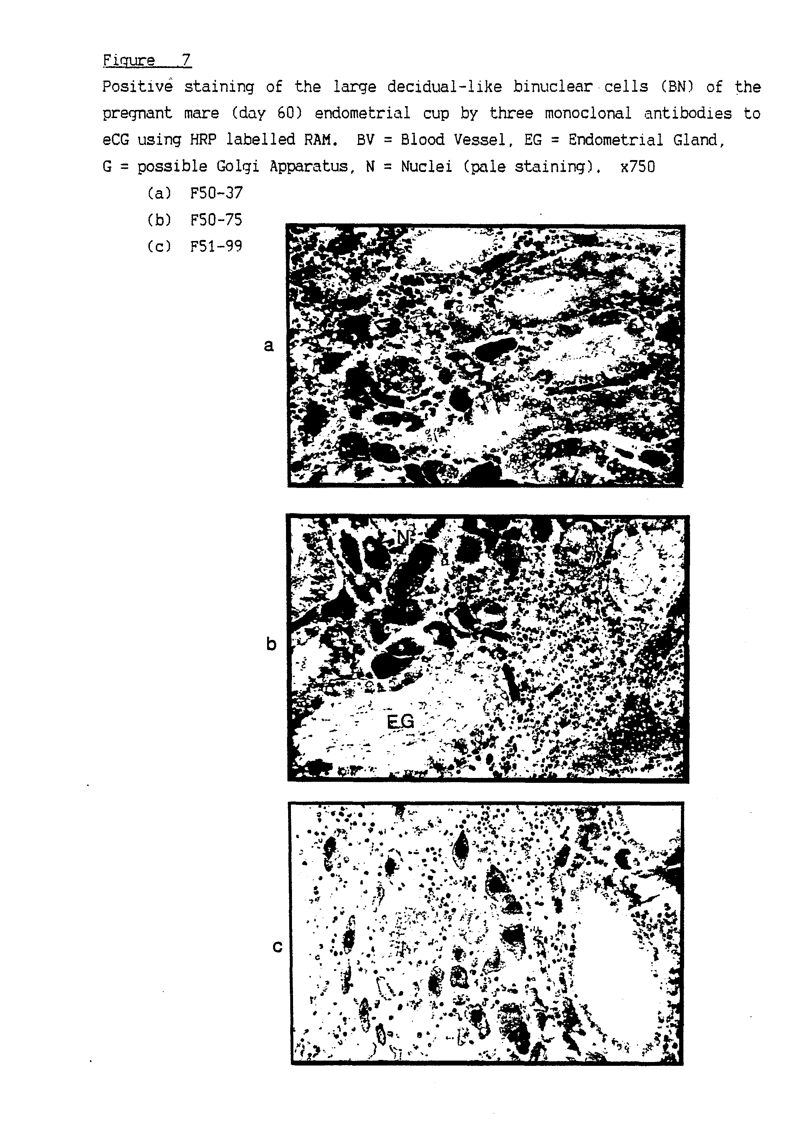

The three eCG monoclonal antibodies were unreactive with human and ovine placental sections, but were strongly reactive with horse endometrial cup sections. Figure 7 shows positive staining of the pregnant mare endometrial cup sections by each eCG monoclonal antibody. The heavy staining in the large binuclear cells of the endometrial cup is probably localised to the golgi apparatus. This could be confirmed using electron microscopy and immunogold staining.

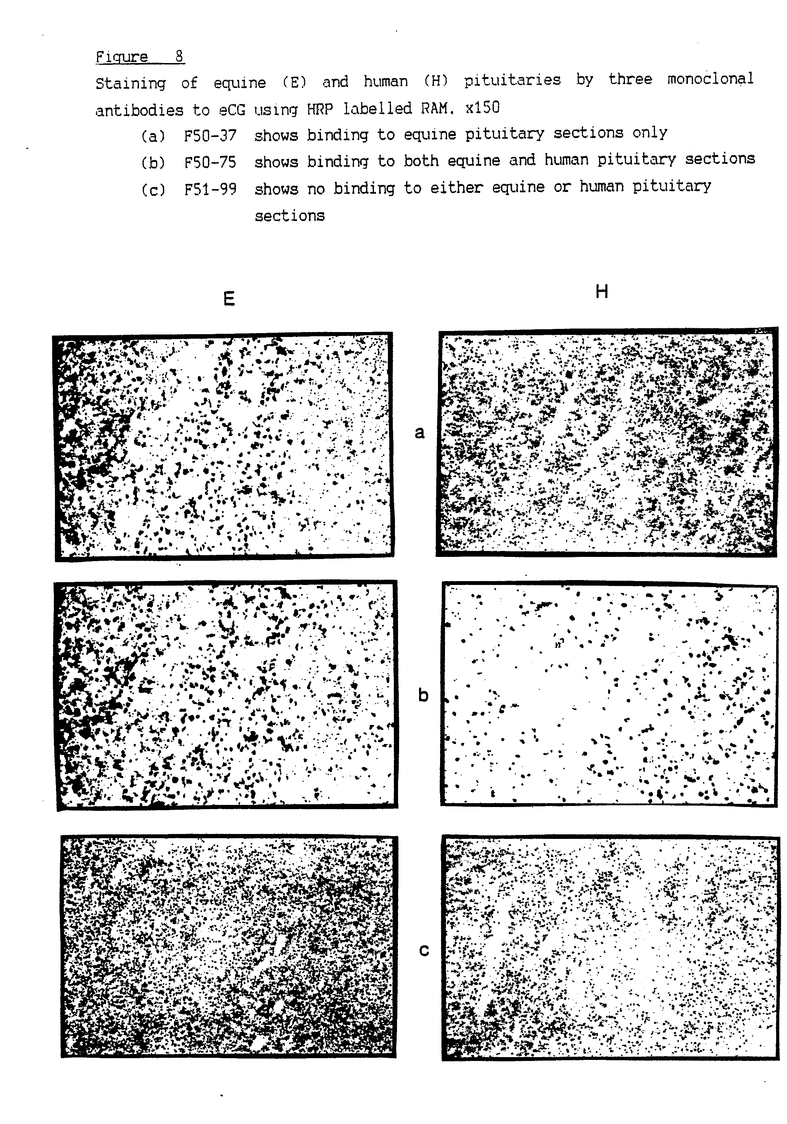

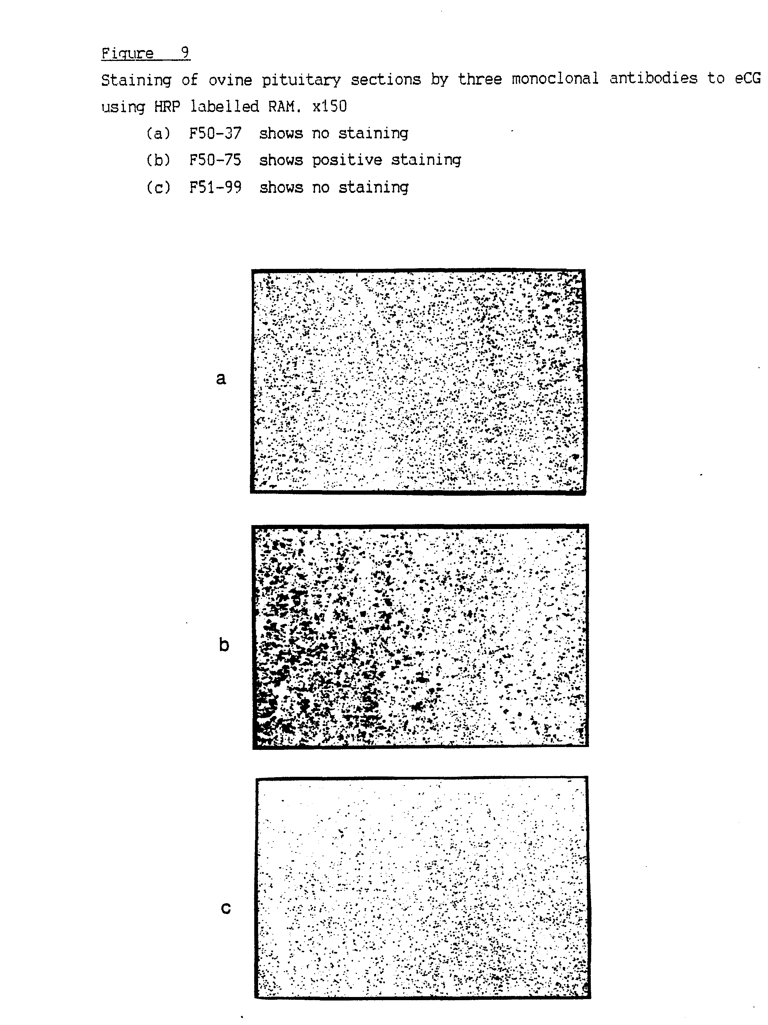

Monoclonal antibody F50-37 showed considerable staining reactivity with scattered cells of the equine pituitary and no specific binding on pituitary sections of the other species examined. Monoclonal antibody F50-75 was reactive with scattered cells of equine, ovine and human pituitaries. Monoclonal antibody F51-99 was unreactive with all pituitary sections. Immunohistological staining of equine and human pituitary sections with the three eCG monoclonal antibodies is shown in Figure 8. Figure 9 shows the staining patterns found for sections of ovine pituitary.

### Additional Hybridoma Cell Lines Producing Monoclonal Antibodies to eCG.

Six further monoclonal antibodies have been established for examination and these are: F65-6, F65-53, F65-88, F65-98, F65-130 and F65-191.

The three most promising, as assessed in agglutination and cross-reactivity studies using uncloned supernatants, have been cloned. These cell lines are F65-88, F65-98 and F65-130. F65-88 and 98 are of the IgM class of immunoglobulin, while F65-130 is an $IgG_1$ class immunoglobulin.

Samples of each of the above mentioned hybridoma cell lines are maintained in the Bunge (Australia) Pty. Ltd. Cell Collection at 89 Flemington Road, North Melbourne, Australia.

### Established uses for the Described Hybridoma Monoclonal Antibodies

### (i) Monoclonal Antibody Affinity Chromatography Purification of eCG

In a still further aspect of the present invention there is provided a method of purifying equine chorionic gonadotrophin (ecG) which method includes providing a source of impure eCG and a monoclonal antibody bound to a matrix; and passing the impure eCG through the monoclonal antibody matrix to capture eCG. The method of purification may further include eluting the captured eCG from the monoclonal antibody matrix.

The monoclonal antibodies bound to the matrix may be any of the monoclonal antibodies described above. Monoclonal antibodies F50-37 and F51-99 are preferred for use in affinity chromatography. F50-37 is preferred because of its high eCG affinity as demonstrated by Scatchard analysis as described below. F51-99 is preferred for its exquisite specificity for eCG.

A simple method for the purification of equine chorionic gonadotrophin is of importance since those methods known in the prior art are both costly and time consuming, and, result in a product of low purity. A purified eCG is of importance as it provides a greatly improved biological response in-vivo. High purity eCG is also extremely desirable for application in human medicine.

The development of technology enabling the purification of eCG through monoclonal antibody affinity chromatography has provided a process whereby the supply of commercial quantities of a highly purified eCG product is possible.

Highly purified eCG has some distinct advantages over the impure eCG products currently available. Batch variation of commercial eCG is extensive and the quality of the in-vivo response is poor by comparison. eCG purified from impure batches of eCG or pregnant mare serum using the presently described technology has little if any batch variation and provides a greatly improved response in-vivo. The improved response is evident in all species tested.

A major advantage of the highly purified product is the reduced incidence of undesirable large follicles which may form in the ovaries adjacent to the Corpora Lutea. The formation of such follicles is implicated in a number of fertilisation complications including restriction of the cervix, blocking of the ovary ducts, excessive oedema and the like. See Table A. By comparing in-vivo responses in the ewe for identical doses of high purity and commercially available eCG, it has been shown that the incidence of unwanted large follicles is greatly reduced in those ewes treated with purified eCG. There was no associated loss or decrease in ovulation rate.

Accordingly, in a still further aspect of the present invention there is provided a purified equine chorionic gonadotrophin (eCG). The purified equine chorionic gonadotrophin is characterised by reduced incidence of large follicles in the ovaries of the animal treated.

TABLE A

In vivo response of the ewe to varying doses of high purity eCG and crude eCG preparations.

| Dose eCG(iu) | Mean No.of Corpora Lutea | | Mean No.of Follicles | |
|---|---|---|---|---|
| | Crude eCG | Purified eCG | Crude eCG | Purified eCG |
| 400 | 2.43 | 2.25 | 1.63 | 0.25 |
| 800 | 3.38 | 3.14 | 2.83 | 0.29 |
| 1200 | 6.38 | 6.75 | 4.50 | 1.25 |
| 1600 | 7.38 | 7.00 | 6.14 | 1.88 |

Negative control animals (animals receiving no eCG) had a mean of 1.43 corpora lutea and 0 follicles N = 200

The matrix used may be of any suitable type. A chromatographic gel is preferred. An affinity chromatographic gel may be used. The gel sold under the trade designation AFFIPREP 10 and available from Bio-Rad Laboratories has been found to be suitable. AFFIPREP 15 is also suitable.

For small scale work and large scale affinity purification of partially purified material, the monoclonal antibody matrix may be contacted with the eCG solution by passing the solution through the antibody matrix supported in a chromatography column. However, it has been found for large scale work with crude material that the monoclonal antibody matrix and the eCG solution may be contacted in large scale containers and vats. The large scale containers or vats may be rotated, agitated or stirred to ensure complete contact between the monoclonal antibody matrix and the eCG solution.

The monoclonal antibody matrix may be provided in any suitable form. The monoclonal antibody matrix may be provided in a solution. A buffer solution may be used. A phosphate buffered saline solution (PBS) may be used.

The source of impure equine chorionic gonadotrophin may be of any suitable type. The eCG source may be pregnant mare plasma or sera. The eCG source may be a crude extract thereof. The eCG source may be in a freeze dried form. The freeze dried sample may be dissolved in a suitable solution. A buffer solution may be used. A phosphate buffer saline solution may be used. It has been found that the pH of the buffer solution may be important in optimising eCG binding. A pH in the range of approximately 6.0 to 7.0 is preferred; more preferably 6.5.

The step of passing the impure eCG through the monoclonal antibody matrix may be undertaken in any suitable container. An affinity column may be used for relatively small scale work. For large scale work, a large container or vat may be used. The large container or vat may be rotated, agitated or stirred to ensure complete contact between the impure eCG and the monoclonal antibody matrix. A rotor bottle may be used as a suitable container.

The captured eCG maybe eluted from the monoclonal antibody matrix utilising any suitable elution solution. An organic acid solution may be used. A pH buffered solution may be used. A sodium chloride-glycine buffer with a pH of approximately 2.0 - 3.0 has been found to provide maximum elution of purified eCG.

Prior to the elution step, the method of purification may further include washing the monoclonal antibody matrix-bound eCG with a washing solution. The washing solution may be similar to the binding buffer.

The elution step may be carried out in any standard affinity chromatography column. A large size column may be used for large scale work.

## (ii) Modification of eCG Activity in vivo

The dual FSH/LH bioactivity and long-half life of eCG in sheep and cattle provides eCG with the potent gonad stimulation effect unique to this hormone. These properties have led to its use in veterinary practice and research for induction of oestrus during anoestrus, increased fecundity and superovulation for embryo transfer.

The larger doses of eCG required for superovulation of livestock often have deleterious effects due to excessive and prolonged over-stimulation of ovarian follicles. The contaminants of known eCG preparations and the extremely long half-life of eCG in these animals results in the formation of large cystic follicles which can cause contractile scar tissue damage to the ovaries and oviducts, and, excessive oestrogen production. These problems greatly reduce the number of quality of recoverable embryos or ova. As a result of the over-stimulation resulting from eCG usage, a means of modifying or controlling eCG bioactivity has been sought. Partial dialysation has been attempted but not fully evaluated Papkoff. [Bio.Chem., Bio-Phy., Acta., 532, 185-194, (1978)]. To date most studies have used polyclonal eCG antisera to modify the half-life of eCG in cattle and sheep. Bindon and Piper [Proc. Embryo transfer in cattle and sheep, Canberra 1981, Published A.S.R.B. (1982)] found that the administration of 2ml sheep anti-eCG serum 24, 48 or 72 hours after eCG injection greatly reduced the superovulatory response of the ewe to eCG. Even at 72 hours, the antiserum effectively halved the mean number of evolutions. The degree of ovarian over-stimulation in each group receiving anti-eCG serum was reportedly reduced but the criteria on which this was established was not presented.

The in-vivo response to eCG has been greatly improved through the application of the following new technologies

(i) High purity eCG, and

(ii) Monoclonal antibodies to eCG.

In a further aspect of the present invention there is provided a method of inducing oestrus and/or increasing fecundity in femalee animals which method includes administering to the animal an effective amount of a purified equine chorionic gonadotrophin. Anoestrus animals are thereby induced to cycle and ovulate. Animals may also show an increase in the frequency of the incidence of associated unwanted large follicles in the ovaries of the animal treated.

The term animals as used herein in the description and claims includes domestic animals including sheep, goats, cattle, pigs, chickens and the like as well as other animals including humans.

For sheep and goats the equine chorionic gonadotrophin may be administered in amounts of from approximately 250 to 1000 iu. For cattle the equine chorionic gonadotrophin may be administered in amounts of from approximately 250 to 1000 iu.

In a further preferred aspect of the present invention there is provided a method of substantially increasing the number of ova produced during a single oestrus cycle in female animals (superovulation). This method includes administering to the animal an effective amount of a purified equine chorionic gonadotrophin. The number of ova produced may be increased to a level of at least about 5 preferably 7. The use of highly purified eCG instead of the current commercially available products greatly reduces the incidence of associated unwanted large follicles in the ovaries of the animal treated.

For sheep and goats the equine chorionic gonadotrophin may be administered in amounts of from approximately 500 to 2000 iu.

For cattle the equine chorionic gonadotrophin may be administered in amounts of from approximately 500 to 3000 iu.

It has now been discovered that monoclonal antibodies to equine chorionic gonadotrophin may block and/or enhance the biological activity of equine chorionic gonadotrophin in animals.

Control of eCG bioactivity in vivo using monoclonal antibodies is advangeous when using eCG for

(i) superovulation,

(ii) increasing fecundity, and

(iii) manipulation of the oestrus cycle.

The advantages already described for superovulation are also applied to the low dose usage of eCG for increasing fecundity, i.e. twinning and manipulation of the oestrus cycles. By using these monoclonal antibodies with low doses of eCG the incidence of twin births in a variety of species can be better controlled. Twinning is therefor easier and more repeatable, and the numbers of unwanted triple and high multiple births are reduced.

Accordingly in a further aspect of the present invention there is provided a method of increasing ovulation to provide twinning in female animals which method includes administering to the animal an effective amount of a purified equine chorionic gonadotrophin characterised in that in use there is a reduced incidence of large follicles in the ovaries of the animal treated.

Preferably the animal to be treated is a sheep, goat or cow and the amount of equine chorionic gonadotrophin administered is in the range of approximately 100 to 1000 iu.

More preferably the method of increasing ovulation to provide twinning further includes administering simultaneously with or after equine chorionic gonadotrophin administration to the animal approximately 0.25 to 1.0 mg of a purified monoclonal antibody against equine chorionic gonadotrophin formed by a hybridoma cell line.

In a preferred aspect of the present invention the method of inducing oestrus and/or increasing fecundity further includes administering to the animal to be treated an effective amount of a monoclonal antibody against equine chorionic gonadotrophin (formed by a hybridoma cell line) simultaneously with or after equine chorionic gonadotrophin administration.

The monoclonal antibody may be any of the antibodies described above. Preferably the monoclonal antibody is monochonal antibody F51-99. The monoclonal antibody may be administered in any suitable effective amount. For sheep and goats the monoclonal antibody may be administered in amounts from approximately 0.25 to 1.0 mg. For cattle the monoclonal antibody may be adminstered in amounts from approximately 1.0 to 3.0 mg. For pigs the monoclonal antibody may be administered in amounts from approximately 0.5 to 1.5 mg.

In the method of super ovulation, the monoclonal antibody may be adminstered for sheep and goats in amounts of from approximately 0.5 to 2.0 mg. For cattle the monoclonal antibody may be administered in amounts of from approximately 0.5 to 3.0 mg.

When administering eCG monoclonal antibodies for control of eCG in-vivo, an accurate assessment of the required dose is essential. Excessive amounts of the monoclonal antibody can lead to an increase in the number of unruptured follicles possibly due to cross-reactive interference with endogenous hormones or stripping of eCG from biological receptors.

The antibody dose suitable is dependent on several variables including:

(i) the monoclonal antibody selected,

(ii) eCG dose,

(iii) time following eCG injection of antibody injection,

(iv) species in which it is being used,

(v) size of the animal, and

(vi) number of times the animal has been previously treated .

An injection of GnRH (gonadotrophin releasing hormone) may also be incorporated into the system. This may be given before antibody administration and about 24 hours before the time of ovulation.

The use of monoclonal antibodies for controlling eCG in vivo whether for use in superovulation, increasing fecundity i.e. twinning or for induction of oestrus as described for ewes and cows has similar application in goats, humans, dogs, cats, pigs and other mammals.

The amount of antibody necessary to be effective will vary with the particular antibody used, the species of animal, the level of eCG treatment and time of administration following eCG injection. For example, where from approximately 1500 to 2000 iu of eCG is injected into a ewe, we have found that approximately 0.25 to 5.0 milligrams of antibody may be used depending on the variables already described. The monoclonal antibody treatment may be undertaken contemporaneously with the eCG treatment or may be undertaken after eCG treatment.

9

Monoclonal antibodies administered simultaneously or after eCG administration can modify the biological activity of eCG. This modification may reduce or enhance the activity of the eCG. Both enhancement and reduction of eCG bioactivity can be incorporated in the same regime.

Preincubation, simultaneous injection or independent administration of eCG and the monoclonal antibody can enhance the activity of the eCG via the formation of antibody-eCG complexes, resulting in greater biological activity.

Equally so the formation of a different antibody-eCG complex can effectively reduce the bioactivity of the eCG or previously complexed eCG by nullifying the biological activity of the eCG.

A trial was undertaken in rats to examine the enhancement properties of 14 eCG monoclonal antibodies. A low (non-effective) dose of eCG was incubated overnight at 4°C with approximately an equal molar amount of each antibody. Pairs of 21-28 day old female PVG rats were then injected with the mixture. 48 hours later rats were examined and scored as for the standard eCG bioassay.

Results

All rats receiving 0.5 iu eCG alone or saline were negative. Identical doses of eCG incubated as described above with monoclonal antibody F65-191 had significant biological activity. This activity was equivalent to the 2.5 iu eCG injected into positive control rats. Monoclonal antibody enhancement of the biological activity of eCG was restricted to monoclonal antibody F65-191, all others tested were negative in this trial. See Table B.

The modification of bioactivity, whether it be enhancement of reduction, of other pharmaceuticals by selected monoclonal antibodies is also possible.

## TABLE B

| Monoclonal Antibody | Unit eCG | Response | |
| --- | --- | --- | --- |
| | | Rat No. 1 | Rat No. 2 |
| Saline only | 0.5 iu | – | – |
| Saline only | 2.5 iu | + | + |
| F50-37 | 0.5 iu | – | – |
| F50-75 | 0.5 iu | – | – |
| F51-99 | 0.5 iu | – | – |
| F65-6 | 0.5 iu | – | –/+ |
| F65-50 | 0.5 iu | – | – |
| F65-53 | 0.5 iu | – | – |
| F65-56 | 0.5 iu | – | – |
| F65-88 | 0.5 iu | – | – |
| F65-98 | 0.5 iu | – | – |
| F65-130 | 0.5 iu | – | – |
| F65-135 | 0.5 iu | – | – |
| F65-146 | 0.5 iu | – | – |
| F65-165 | 0.5 iu | – | – |
| F65-191 | 0.5 iu | + | + |

### (iii) eCG Detection and Quantitive Assays

In a still further aspect of the present invention there is provided a method for the detection of equine chorionic gonadotrophin (eCG) in an equine mammal. The detection assay may be an immunofluorescent assay, radioimmuno-assay, enzyme-linked immuno assay or agglutination assay. For the IgM immunoglobulin class monoclonal antibodies an agglutination assay is preferred. A haemagglutination assay is preferred.

In a particularly preferred form the detection assay is a haemagglutination inhibition assay.

For the IgG immunoglobulin class monoclonal antibodies, the RIA and ELISA assays are preferred.

The sample to be tested may be of any suitable type. Sera, plasma, saliva or urine samples or extracts thereof may be used.

Thus, in a preferred aspect, there is provided a method for detecting the presence of equine chorionic gonadotrophin, which method includes providing a sample from the equine mammal to be tested, an IgM class eCG monoclonal antibody from those described above, or mixtures thereof and a source of equine chorionic gonadotrophin (eCG)-coated mammalian red blood cells. Contacting the monoclonal antibody/s with a portion of the sample to bind any equine chorionic gonadotrophin present; and contacting the mixture of antibody and sample with the eCG-coated mammalian red blood cells.

It would be understood that the presence of eCG may then be detected by visual means. Where eCG is present in the serum of interest, the eCG will bind to available binding sites on the monoclonal antibodies. When the eCG coated mammalian red blood cells are added there is effectively no antibody to bind to the antigen on the cells and there is a resultant inhibition of agglutination. Non-agglutinated cells subsequently slide around the round bottom of the solid support well to form a dark dot of cells.

The solid support may be of any suitable type. A microtitre plate or tray or test tube may be used. A microtitre plate or tray of the round bottom type is preferred. The monoclonal antibodies may be introduced as a solution. A phosphate buffered saline solution may be used.

The equine chorionic gonadotrophin coated mammalian red blood cells may be eCG coated sheep red blood cells.

In a preferred form the detection methods may be used for detecting other pregnancy proteins in other mammals. The detection method is particularly applicable to eCG in equine animals, although hCG in humans and other pregnancy proteins in other mammals may be similarly tested.

Thus in an alternative form, a human chorionic gonadotrophin-specific monoclonal antibody may be incorporated in any suitable assay to detect human chorionic gonadotrophin.

Thus in a preferred alternative aspect, there is provided a method for detecting the presence of human chorionic gonadotrophin, which method includes providing a sample from the human to be tested, an hCG - (human chorionic gonadotrophin) - specific monoclonal antibody, or mixtures thereof an a source of hCG - coated mammalian red blood cells, contacting the monoclonal antibody with a portion of the sample to bind any hCG present; and contacting the mixture of antibody and sample with the hCG - coated mammalian red blood cells.

The hCG-specific monoclonal antibodies may be elected utilizing similar immunohistological techniques to those described above for selecting eCG-specific monoclonal antibodies.

Accordingly, in a further aspect of the present invention there is provided a test kit including
a source of monoclonal antibody in a suitable container;
a source of eCG-coated mammalian red blood cells in a suitable container;
and a solid support.

The monoclonal antibodies may be provided in a solution. A buffered solution may be used.

The eCG coated mammalian red blood cells may be eCG coated sheep red blood cells.

The solid support may be a plastic support. The solid support may be microtitre plate or tray or test tube. A round bottomed microtitre plate or tray may be used. The solid support may take the form of plastic balls, paddles, tubes or membranes. The solid support may be in the form of a glass. Glass beads may be used. The solid support may be in the form of a paper. A filter paper or paper membrane may be used.

The established eCG specificity of monoclonal antibody F51-99 is ideally suited for the development of an eCG specific assay. The mono and/or bivalency of this class of immunoglobulin (IgG) usually renders it unsuitable for agglutination type assays but suitable for ELISA and RIA systems. The ELISA system is well suited for "in-field" eCG detection. In such a system enzyme conjugated F51-99 is mixed in solution with the sample in question. If sufficient eCG is present the labelled antibody is "mopped up", if not some remains free in the mixture of solutions. This free antibody is then removed for detection. This is accomplished by exposing this solution (mix) with immobilized eCG. Unblocked or free antibody remaining in the solution then binds. Residual unbound complexes are rinsed away from the antibody bound to the immobilised eCG. Simple exposure of the eCG matrix, with or without antibody attached, to a substate solution determines the presence or absence of antibody through eg. a colour change reaction.

The reserse ELISA system to that described here is also applicable where the antibody is immobilised and the eCG is labelled with an enzyme.

Enzymes suitable for coupling to the monoclonal antibody and eCG include urease, horse radish perioxidase and alkaline phosphatase. Each has its own relevant substrate system and colour change reaction as known in the art.

Accordingly, in a further aspect the present invention provides a veterinary or pharmaceutical composition for the modification of the biological activity of eCG in mammals including an effective amount of a monoclonal antibody selected from those described above or mixtures thereof in a unit dosage form.

The veterinary or pharmaceutical composition may further include a veterinarily acceptable or pharmaceutically acceptable diluent carrier or excipient therefor. The monoclonal antibody may be provided in solution. A buffered saline solution may be used. A phosphate buffered solution may be used.

In an alternative aspect, there is provided a method of inducing abortion in pregnant mammals, which method includes administering to the mammal an effective amount of a monoclonal antibody selected from those described above or mixtures thereof.

The method of inducing abortion of unwanted pregnancies is particularly applicable to equine animals. This is particularly applicable in relation to thoroughbred horses, where abortions are required for mares carrying twins or foetuses of unknown paternity. The treatment method is in general similar to that used for blocking of the biological activity of eCG. However, in general larger dosages may be required.

The present invention will now be more fully described with reference to the accompanying examples. It should be understood, however, that the description following is illustrative only and should not be taken in any way as a restriction on the generality of the invention described above.


EXAMPLES

MATERIALS AND METHODS

Serum Collection and Crude Extract Preparation

Sera were collected fresh at a local knackery. Twelve ml of 10% potassium oxalate was added to each litre of whole blood as an anticoagulant. Supernatants were collected after settling and frozen at -20°C until required for processing.

Extractions from sera using the technique of Gospadorowicz and Papkoff (1967) gave poor eCG yields (5-10%). The technique was subsequently altered as follows. 0.25M metaphosphoric acid was freshly prepared at 4°C and added to cold serum with stirring until a pH 3.5 was reached. After centrifugation (15,000g for 30 minutes at 4°C), the supernatant was poured off and adjusted to pH 4.5 with 1.0M sodium hydroxide. The supernatant was then made up to a 40% solution (v/v) with ethanol by slow addition of absolute ethanol (-20°C) while stirring. The supernatant was then removed by centrifugation (as above) and made up to a 75% solution (v/v) with absolute ethanol (-20°C) while stirring. The resulting precipitate was harvested by centrifugation, dialysed against double distilled water, freeze-dried and stored at -20°C until required.


eCG:Haemagglutination Inhibition Assay

All procedures were performed at room temperature and the haemagglutination inhibition assay was based on reagents supplied in a commercially available mare pregnancy diagnosis kit having the trade name of "M.I.P. (Mare Immunological Pregnancy) Test Kit" (Carter-Wallace, Inc., N.J., USA). The eCG standard for the assay was the W.H.O. 2nd International Standard for PMSG (W.H.O., London).

eCG test samples and the eCG standard were titrated by doubling dilution in 50ul volumes of phosphate buffered saline containing 0.1% sodium azide (PBS/Az) in 96 round well titration trays (Flow Laboratories). The following solutions were added per well as directed by the manufacturer, to eliminate possible non-specific agglutination during the assay. 5ul of M.I.P. Test Kit Inhibitor solution was added and mixed by rocking for 90 seconds. 5ul of M.I.P. Test Kit Neutraliser solution was then quickly added. 15ul of M.I.P. Test Kit Antisera solution, (the optimal dilution being determined by titration to give sufficient agglutination of 10ul of Test Kit eCG coated Sheep Red Blood Cells, [eCG SRBC Solution]), was then aded and mixed by occasional rocking for 5 minutes. 10ul of M.I.P. Test Kit eCG SRBC Solution was then added per well and the tray rapidly rotated on a Microshaker (Cat. No. Am69, Dynatech, England) for 10 seconds to ensure good cell distribution prior to settling. End points for the assay as determined by red blood cell settling patterns, ranged from 0.5iu/ml - 4iu/ml depending on the batch of the kit used. To standardise with the values obtained using the rat ovarian weight bioassay as described by Cole and Erway, Endocrinology 29 (1941).

## ELISA assay of eCG

ELISA (96 well) plates were coated with 50 ul of 8 ug/ml monoclonal antibody F50-37, 6 ug/ml F50-75 in 0.1 M carbonate buffer pH 9 for 60 minutes at 37°C in a humidified chamber. Mixtures of other monoclonal antibodies were also successfully used.

The coating solution was then aspirated and 250 ul of blotto (4% skim milk in PBS) added to each well. This was again incubated as above.

eCG samples were titrated by doubling dilution in blotto in a separate 96 well plate. After aspiration of the blocking solution and 3 distilled water washed titrated samples were then transferred to the coated ELISA trays. The trays were incubated for 60 minutes as above.

Urease conjugated monoclonal antibody F51-99 diluted 1:100 in blotto was added to each well after thorough washing of the ELISA tray and incubated as above for a further 60 minutes.

After a final 3 water washes 75 ul of urease substrate solution was added per well and the reaction allowed to proceed until background reactions began to appear. Trays were then read on a Multiscan Titretek ELISA reader and eCG concentrations determined by extrapolation from a standard curve. Folligon 100 iu/vial material was incorporated as the standard.

## Bioassay of eCG

Pairs of 21-26 day old virgin female PVG rats were lightly anaesthetized with chloroform and injected intraperitonealy (IP) with 0.2ml of each dilution of a standard or test sample of eCG in PBS. Autopsies were performed 48 hours after injection and ovarian/uterine development, including size and vascularization, assessed and scored on a 4 point scale as " + + ", " + ", " + - " or "-". some variation in end points was found between assays, but typically an injection of 4iu or more gave a " + + " result, 2iu " + ", liu " + -" and 0.5iu a "-" result. This assay was adapted from that described by Cole and Erway [Endocrinology, 29, 514-519 (1941)].

## Production of monoclonal antibodies to eCG

IMMUNIZATION:

Four BALB/c mice were injected IP with 1,000 iu eCG dissolved in 100ul PBS and emulsified in a further 150ul of Freund's Complete Adjuvant (FCA). The same batch and quantity of eCG in Freund's Incomplete Adjuvant (FIA) was injected IP weekly for a further 3 weeks. One week after the final injection, mouse sera obtained by tail bleeding were examined for eCG reactivity and specificity by immunohistology on a range of pregnant and non-pregnant mare tissues. Mice having the highest serum titres were boosted 4 days prior to fusion with 1,000iu eCG in 0.1ml PBS by tail vein injection.

FUSION AND CLONING:

The following reagents were] required for the production of monoclonal antibodies and were prepared as follows:

(i) GKN Solution: 8.0g NaCl, 0.4g KCl, 1.42g $Na_2HPO_4$ $2H_2O$, 2.0g D-Glucose and 0.01 g phenol red were dissolved per litre of double distilled water, filter sterilized and stored at room temperature.

(ii) Tissue culture media: Dulbecco's Modified Eagles medium (DME, powdered and sodium bicarbonate free, Gibco USA) and sodium bicarbonate (0.2% w/v) were dissolved in double distilled water as directed, filter sterilized and stored at 4°C. Prior to use, the DME solution was supplemented with 10% - (v/v) foetal calf serum (FCS, Flow Laboratories) and 2mM L-glutamine (200mM L-glutamine, Flow Laboratories). HAT selective media contained a further 13.6mg hypoxanthine, 0.19mg aminopterin and 3.87mg thymidine per litre of media. HT selective media was identical to HAT selective media except it contained no aminopterin.

Fusion and cloning steps were carried out as follows: Briefly, a primed mouse was killed by cervical dislocation and the spleen aseptically removed. Splenocytes were obtained by gentle squeezing and teasing of the spleen in 10ml GKN solution. The cell suspension was allowed a short settling period (30-60 seconds) to permit the removal of large clumps of tissue. The cells remaining in solution were washed twice by centrifugation (200 g for 5 minutes at room temperature) and resuspensed in 10ml GKN. After the final centrifugation the cells were resuspended in a further 10ml GKN.

NS-1 myeloma cells, 2 x $10^7$, (P3/NSI/1-Ag4-1) grown in tissue culture media were collected by centrifugation (200 g for 5 minutes at room temperature) and resuspended in 10ml GKN solution.

The 10mls of splenocytes and NS-1 myeloma cells were mixed in a 50ml centrifuge tube (Falcon) and centrifuged at 70g for 5 minutes at room temperature. The supernatant was then poured off to leave a pellet of cells which were resuspended to form a slurry, by gently tapping the tube, in a minimum of residual supernatant. 1.5ml of sterile 50% polyethylene-glycol at 37°C [PEG, Art. 9727 Polyethyleneglycol 4000, Merck] prepared by autoclaving 1.0g PEG with 1ml double distilled water in a sealed container) was then added to the slurry over 90 seconds, with gentle mixing using the pipette as a stirring rod. After a further 30 seconds the mixing, GKN at 37°C was carefully added with stirring. The first 2ml of GKN was added over 2 minutes, and then a further 8ml was added over 3 minutes. Another 30ml GKN was then gently added down the side of the centrifuge tube.

The cell suspension was immediately centrifuged at room temperature for 3 minutes at 70g with the brake off, and the supernatant poured off. The tube was tapped gently to form a slurry of cells before resuspension in 50ml of HAT selective media. 0.5ml samples of the cell suspension were then added to each well of four 24 well flat bottom Linbro plates (Flow Laboratories) each containing 1.5 x $10^6$ rat thymocytes in 1.5ml HAT selective media. The thymocytes were prepared on the day prior to the fusion and incubated overnight at 37% in a humidified 5% $CO_2$ atmosphere to ensure sterility.

Hybrid colonies were monitored using an inverted microscope (Olympus). 10-11 days after fusion supernatants were screened by ELISA and immunohistology. Selected wells were dilution cloned in 96 well flat bottom Linbro plates (Flow Laboratories) with thymocytes in HAT selective media. Three trays containing approximately 1, 10 and 50 hybridoma cells per well were prepared.

ELISA Screening Assay

96 well flat bottom Linbro plates (Flow Laboratories) were coated with antigen by incubating overnight at 4°C with 50ul/well of a solution containing 15-120ug/ml of protein in 0.5M $Na_2HPO_4$, pH 9 containing 0.1% $NaN_3$. Trays were rinsed 3 times with PBS prior to use. Sera and supernatants were diluted 1:500 and 1:1 in "blotto" (4% skim milk powder [Diploma, Australia], in PBS and made fresh daily) respectively for screening. 50ul volumes of diluted antibody were added per well and the trays then incubated for 2-3 hours at room temperature in a humidified box.

Following three rinses with PBS, 50ul of peroxidase-conjugated rabbit anti-mouse immunoglobulin (RAM-HRP, Dako, Denmark) diluted 1:1,000 in blotto was added per well and the tray incubated for 1-2 hours as above. After a further 3 rinses with PBS, 100ul of substrate solution was added per well. This solution was prepared fresh on the day of use by dissolving 40mg 0-phenyline diamine (BDH) in 100ml 0.01M citrate buffer pH 5.0. Immediately prior to use, 80ul of 30% hydrogen peroxide was added with stirring.

The reaction was stopped within 30 minutes by the addition of 25ul of 25% (v/v) sulphuric acid per well, depending on development of the background wells. Trays were read on a Microtitre Multiskan (MCC, Finland) at 492nm.

## Immunohistology

Fresh tissues were collected at a local knackery and fixed in Bouin's solution for 2 hours, or in cold 95% ethanol overnight. After trimming, tissues were embedded in paraffin by processing through four 1 hour changes of absolute ethanol at 4°C , four changes of xylene at room temperature, and four changes of liquified paraffin at 56°C, and then left to solidify overnight at 4°C.

Tissue sections of 3-5um were cut using a microtome (Leitz) and floated onto glass slides from a warm 1% gelatin (w/v) water bath. Slides were then incubated overnight at 37°C to adhere the sections. Prior to use, sections were rehydrated by 5 minute incubations in each of the following solutions; xylene, fresh (wax free) xylene, absolute ethanol, fresh (xylene free) absolute ethanol, 75% ethanol and running tap water. Thorough paraffin removal was found to be essential to minimize background staining due to non-specific binding of the second antibody. After two rinses in double distilled water, sections were kept moist and used within 2 hours.

Neat hybridoma supernatants,or mouse sera diluted 1:100 in blotto were placed directly onto the sections and the slides incubated in a humidified box for 2-3 hours at room temperature. After gentle rinsing with PBS, sections were covered with rabbit anti mouse IgG conjugated to horse radish peroxidase (RAM-HRP) diluted 1:60 in blotto and incubated for a further 1-2 hours as before. After thorough but gentle rinsing, the slides were submerged in a substrate solution containing 0.14g 3,3'-diaminobenzidine tetrachloride in 250ml 0.01M citrate buffer, pH 5, plus 250ul of 30% hydrogen peroxide added immediately prior to use. Within 10 minutes the reaction was stopped by rinsing in tap water.

Sections were counterstained with Harris' haematoxylin and lithium carbonate, and then dehydrated by 5 minute rinses through 70% ethanol, absolute ethanol, xylene and fresh xylene for mounting with coverslips and Depex.

## Chloramine T Radiolabelling of eCG

1mCi $^{125}$I (100mCi/ml, Amersham) was mixed with 10ul 2mg/ml chloramine T in 0.3M sodium phosphate pH 7.4 (freshly prepared) and 10-20ul 1mg/ml eCG in Dulbecco's A + B buffer plus 0.1% sodium azide (DAB/Az) for 10-20 seconds at room temperature. The reaction was stopped by the addition of 25ul saturated L-tyrosine solution. 100ul 10% BSA in DAB/Az was then added to the mixture prior to loading onto a 5ml G-50 Sephadex (medium grade) column packed in a disposable 10ml pipette. Prior to separation the column was washed with 250ul 10% BSA in DAB /Az and equilibrated with DAB/Az.

The loaded sample was eluted with DAB/Az and collected as three 1.5ml fractions. Fraction 2 contained the labelled protein while free $^{125}$I was eluted late in fraction 3, as determined by 10% trichloroacetic acid precipitation of the fractions.

## Sodium Dodecyl Sulphate Polyacrylamide Gel Electrophoresis (SDS-PAGE)

SDS-PAGE analysis was based on the method of Laemmli [Nature, 227, 680-685, (1970)] and used the following reagents:

(A) acrylamide/bisacrylamide (Bio-Rad) 37.5:1. A stock solution of 30.8g/100 ml of double distilled water was stored at 4°C in the dark.

(B) 0.75M Tris-HCl, pH 8.8

(C) 1.0M Tris-HCl, pH 6.8

(D) 10% SDS in double distilled water

(E) 1% (w/v) ammonium persulphate

(F) TEMED (Sigma)

10% acrylamide gels were prepared by mixing 10ml of (A), 15ml of (B), 0.3ml of (D), 3.2ml of double distilled water, 1.5ml of (E) and 0.02ml of (F). (E) and (F) were added just prior to pouring of the gel. The stacker gel was prepared with 0.8ml of (A), 1.0ml of (C), 0.08ml of (D), 4.7ml of double distilled water, 0.4ml of (E) and 0.012ml of (F). Again (E) and (F) were added immediately before use.

Gels were run over 4-5 hours at 160V (constant voltage) using a Bio-Rad Protean cell kit and running buffer consisting of 0.025M Tris, 0.192M glycine and 0.1% SDS.

Samples were prepared by incubating at room temperature for 30 minutss or by boiling for 5 minutes in an equal volume of sample buffer. Sample buffer was prepared by mixing 10ul 5% bromophenol blue, 200ul glycerol, 250ul 20% SDS and 500ul 1.0M Tris-HCl pH 6.8. Reducing sample buffer contained in additional 4% dithiothreitol.

Coomassie blue staining of gels was performed by overnight immersion in a 45.5% methanol, 9% glacial acetic acid solution containing 0.125% (w/v) Coomassie blue. Gels were destained by overnight rocking in a 25% methanol, 7.5% glacial acetic acid destain solution.

Molecular weight markers used were (Cat. No. SDS-7, Sigma); $M_r$ 14,200 $\alpha$-lactalbumin, $M_r$ 20,100 soyabean trypsin inhibitor, $M_r$ 24,000 trypsinogen, $M_r$ 29,000 carbonic anhydrase, $M_r$ 36,000 glyceraldehyde-3-phosphate dehydrogenase, $M_r$ 45,000 egg albumin and $M_r$ 66,000 bovine albumin.

## Western Immunoblotting

SDS-PAGE gels were soaked for 15 minutes in Western transfer running buffer (0.25M Tris, 0.192M glycine, 20% methanol) and loaded into a Bio-Rad Transblot apparatus as recommended by the manufacturer. Transfer of proteins onto the nitro-cellulose paper (0.1um) was performed overnight at 4°C using a current of 150mA. The nitrocellulose paper was removed and blocked by incubation in blotto for 1 hour at room temperature. After three rinses in PBS/Az the paper was cut and placed into resealable plastic bags containing 20-30ml of monoclonal antibody supernatant. The bags were sealed and rocked for 2-3 hours at room temperature on a Bellco rocking platform (Cat. No. 7740-20220 Bellco, USA) prior to thorough rinsing with PBS/Az and the addition of 20ml RAM-HRP second antibody diluted 1:500 in blotto. After 1-2 hours incubation at room temperature while rocking, and thorough rinsing in PBS (no azide), the colour reaction was developed as described for immunoperioxidase staining of tissue sections.

## Isotyping

Monoclonal antibodies supernatants were isotyped using a dot-blot technique. Reagents were purchased as an immunoglobulin class and subclass detection kit (Chemicon International, USA) and used according to the manufacturer's instructions. An alternative second antibody to that provided in the kit, a peroxidase conjugated goat anti-rabbit IgG (Lot No. LP002) was purchased from the same company and diluted 1:500 for use.

## Ascites Production and Purification of Mouse IgG

### (i) Production:

Cloned hybridoma cell lines were slowly taken off HAT selective media via HT media into HAT free media. $1 \times 10^7$ hybridoma cells were washed three times in PBS by centrifugation, and then resuspended in 0.5ml PBS for IP injection. Recipient BALB/c mice were injected with 0.5ml pristane (2, 6, 10, 14-tetramethyl pentadecane 96%) 3 weeks prior to hybridoma cell injection.

Following cell injection mice were regularly observed and killed when sufficient ascites was present. The ascites fluid was centrifuged (240g for 5 minutes at room temperature) and the supernatant removed. Sodium azide was then added to a final concentration of 0.1%, and the ascites fluid stored at 0-4°C for periods of less than 2 days, or at -20°C for longer periods, or at -70°C without azide.

### (ii) Purification:

Ammonium sulphate precipitation and DE-52 chromatography was used to extract and purify immunoglobulin from ascites fluid. Equal volumes of ascites fluid and saturated ammonium sulphate solution were mixed by dropwise addition of the latter, with rapid stirring. The mixture was left to stand for 1 hour at room temperature and then centrifuged (12,000g for 20 minutes at 20°C). The pellet was washed twice by resuspension and centrifugation in 50% saturated ammonium sulphate. The precipitate was then redissolved in double distilled water and dialysed extensively against 25mM Tris, 25mM NaCl, pH 7.4.

A 50ml DE-52 Cellulose (Whatman, Kent England) column was packed in a 50 ml disposable syringe (Terumo, Aust.) and extensively equilibrated with the 25mM NaCl, 25MM Tris pH 7.4 starting buffer. When the dialysed sample and column effluent conductivities, as measured on a conductivity meter (Model 101, Orion) were ± 1-2mS, the sample was pumped onto the column at 40ml/hr, and 10ml fractions collected on a LKB fraction collector with a LKB UV spectrometer and chart recorded. All chromatographical procedures were performed at 4°C.

Once the elution profile had returned to baseline levels, following thorough washing with the starting buffer, the column was eluted sequentially with 50mM, 100mM, 150mM and 200mM NaCl. Each eluted peak was then examined for activity by ELISA assay and the relevant peak pooled and made up to 0.1% $NaN_3$ for storage at 0-4°C, short term, or -20°C for longer periods.

Production of Rabbit Antiserum to Mouse IgG

Rabbit anti-mouse IgG was prepared by 4 fortnightly intramuscular injections of 1mg purified mouse IgG (2mg/ml PBS) emulsified in 1ml Freund's complete adjucant (primary immunization only) or Freund's imcomplete adjuvant. Rabbits were bled one week after the final immunization.

Preparation of Affinity Chromatography Columns

Purified immunoglobulin from ascites fluid was coupled to AFFIGEL 10 (Biorad) following dialysis in 0.1 M $NaHCO_3$ pH 8.5, according to the manufacturers instructions. One volume of gel was coupled at 4°C with 2-3 volumes of antibody solution (16 mg/ml) by rotational mixing overnight.

Photography

Agfapan 25 film (Agfa-Gevaert) was used for photography of Western immunoblots. The film was developed by Rodinal (1/25) for 5 minutes at 18-21°C, washed in tap water, and then fixed for 5 minutes in rapid fixer A (Kodak). The negatives were printed on grade 3 paper (Kodak) using Dektol developer (Kodak).

Agrachrome 50L film (Agfa Gevaert) was used for colour photography of immunohistological slides. Colour film was printed using the Ektaflex process (Kodak). Magnifications cited for colour illustrations have been corrected for magnification during photography and printing.

EXAMPLE 1

The Production and Characteristics of Monoclonal Antibodies to eCG

Screening antigens:

Three serum batches were processed according to the adapted extraction technique of Gospodarowicz, endocrinology 80 (1967) described in Chapter 2. These were,
    (i) 1.0 l of serum from a day 60 pregnant mare (PMS)
    (ii) 0.5 l normal mare serum (NMS), and
    (iii) 0.5 l normal stallion serum (NSS).
All crude preparations were dialysed against double distilled water, freeze dried and stored at -20°C. Further purification of 73mg of the crude PMS preparation was undertaken according to the method described by Gospodarowicz (1967) using Sephadex G-100 gel-filtration. This resulted in a 4 fold increase in specific activity from 400iu/mg to 1600iu/mg.

Immunogens:

Three different eCG preparations were used for immunization,
    (i) 1,600iu/mg material prepared as above.
    (ii) 10,000iu/mg pure eCG (NIH).

(iii) 400 iu/mg "Twinject PMSG" (Batch 8367, Immunoproducts Melbourne)

<u>Screening assays</u>:

Two screening techniques were used; enzyme linked immunosorbent assay (ELISA) and immunohistology. Immune sera and hybridoma supernatants were screened by ELISA assay as described above using PMS and NMS coated 96 well flat bottomed PVC trays. Supernatants which gave positive binding to PMS coated wells and no binding to NMS coated wells were further examined by immunohistology on 5um sections of pregnant (day 60) and non-pregnant mare uteri. Antibody preparations staining specifically in tissues known to synthesise eCG were selected for further study.

<u>Cell fusions</u>:

Two fusions were performed one week apart according to the method described above and were code named F50 and F51 respectively. Supernatants from the hybridoma cell lines were screened 10-12 days after fusion. Hybridomas of interest were dilution cloned at least 4 times to assure the selection of a single cell line.

Of the 12 eCG immunized mouse sera screened, 4 gave strong positive staining by immunohistology on endometrial cup sections, 6 gave weak staining and 2 gave no staining at all. The groups of mice immunized with the NIH and 1600iu/mg eCG preparations each contained 2 mice with strong positive sera. The positive sera of the 2 mice injected with the NIH preparation had a higher titre, and stained more specifically in endometrial cup tissues. These mice were chosen for the production of hybridomas.

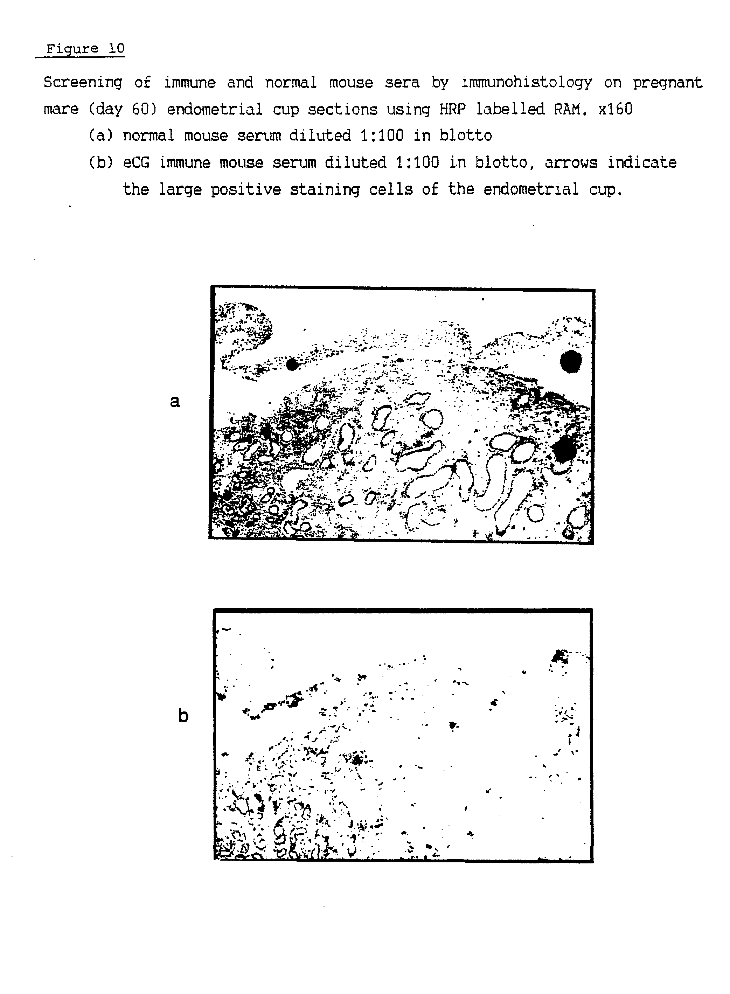

Normal and immune mouse sera staining of day 60 endometrial cup sections from a 60 day pregnant mare uterus are compared in Figure 10. The large cells evident in the centre of the negative control section 10(a), which are not stained, are clearly immuno-reactive with the immune mouse serum in 10(b) as shown by the strong brown reaction product. Some cells associated with the overlying foetal chorion also showed positive staining. If staining is specific for eCG, this may be due to local eCG production or the adherence of eCG secreted by the large uterine embedded decidual-like cells of the cup tissue through the glandular openings into the lumen. The tissue seen in the bottom left corner of Figure 10(b) shows very little if any staining, and is representative of staining by this antiserum on non-pregnant uterine tissue sections.

Fusion 50 produced 12 wells with PMS-reactive supernatants by ELISA assay, out of the 192 wells containing hybridomas. Of these 12 supernatants 10 were also NMS-reactive. The specificity of each supernatant was further checked using immunohistology and showed that the same 2 wells specific for PMS by ELISA assay, wells 37 and 75, were specific for endometrial cup tissue. Other supernatants stained non-specifically or not at all. Wells 37 and 75 were dilution cloned at least 4 times prior to coding the cell lines F50-37 and F50-75 respectively.

Fusion 51 produced only one PMS-positive, NMS-negative well, by ELISA and immunohistology, well 99. This was dilution cloned 4 times and coded F51-99.

Large quantities of high titre supernatant were produced for all three cell lines. Uncloned wells from the initial 24 well trays were pooled, expanded and frozen for storage in liquid nitrogen. Cell lines derived at each dilution cloning were also expanded and frozen in liquid nitrogen.

The ELISA screening assay was designed to select for monoclonal antibodies to antigens present in crude extracts of PMS and absent in NMS extracts. This assay did not exclude the possibility of selecting for antibodies directed against other pregnancy related antigens in the PMS crude extract. Further screening using immunohistology restricted the specificity of the selected antibodies to those molecules found in endometrial cups of pregnant mares. By screening on a variety of endometrial cup sections originating from several different mares, the possibility of selecting for antibodies to paternally derived antigens expressed on the surface of the endometrial cup cells was eliminated.

Further studies were undertaken to demonstrate the reactivity of the three monoclonal antibodies with purified eCG.

Although the screening procedures used in the identification and subsequent cloning of hybridomas were selective for pregnancy related antigens localised to the endometrial cups of pregnant mares, conclusive eCG reactivity of each monoclonal antibody had not been demonstrated. To establish this, radio-immunoprecipitation experiments were performed to show binding of $^{125}$I labelled eCG by each monoclonal antibody. Once the eCG reactivity of each monoclonal antibody had been established, further studies to enable Scatchard plot analysis [Scatchard, Ann. N.Y. Acad. Sci.. 51, 660-672, (1949)] and comparison of the affinities for the three eCG monoclonal antibodies were undertaken.

Prior to commencing the Scatchard plot analysis, a preliminary examination of the valency of each monoclonal antibody was made to provide an important insight into the binding capacities of each antibody. This included an examination of monoclonal antibody isotypes and their ability to agglutinate eCG coated SRBC.

Once the eCG reactivity of each monoclonal antibody had been established, further experiments to classify the specificity of each were undertaken. All previously reported eCG antisera when tested have shown considerable cross-reactivity with the equine pituitary gonadotrophins LH and FSH.

To examine the specificities of the three eCG monoclonal antibodies, supernatants were examined by immunohistology for cross-reactive binding on sections of placenta and pituitary from horses, sheep and man. Sections of human placental tissue previously shown to contain large quantities of hCG by immunohistology with a polyclonal hCG antiserum, were used as representative sections of human placenta.

## Materials and Methods

### Radio-Immunoprecipitation of $^{125}$I eCG:

5ug of pure eCG (UCB Bioproducts, Belgium) was reacted with 0.5mCi $^{125}$I (100mg/ml $^{125}$I, Amersham) using the chloramine T method. 0.5ml samples of each eCG monoclonal supernatant and one non-associated negative control supernatant were titrated by doubling dilution from neat to 1:128 in PBS/Az. 30,000 cpm $^{125}$I eCG in 100ul PBS/Az containing 1.25% normal BALB/c mouse serum, was added to each tube and incubated for 2.5 hours at room temperature (20-25°C). 10ul of RAM was added and incubated for a further 1 hour at room temperature. Supernatants were removed by centrifugation (3000g for 30 minutes at 4°C) and the immunoprecipitates counted using a Packard gamma counter (Model 500C, Packard Illinois, USA).

### Standard curve construction:

30ug of pure eCG (UCB, Belgium) was labelled with 1mCI $^{125}$I by enzymatic (lactoperoxidase) iodination using Enzymobeads (Bio-Rad) as described in Bio-Rad Technical Bulletin No. 1071. Iodinated eCG was separated from reagent beads and free $^{125}$I by G-50 Sephadex gel-filtration as described for the chloramine T iodination method. Antibody supernatants were concentrated (10x) using an Amicon Diaflo filtration system (Model 202) and Amicon PM30 filter (Amicon, Massachusetts, USA).

Several separation schemes incorporating Protein A Sepharose (Pharmacia), RAM and polyethylene glycol were investigated and the scheme giving the best results and repeatability used for the construction of the antibody dilution curves.

0.5ml aliquots of 10x concentrated supernatants were titrated by doubling dilution from 5x conc. to 0.00019x conc. in PBS/Az containing 2% foetal calf serum (FCS). The FCS was added to reduce background in tubes containing low concentrations of antibody due to non-specific adherence of $^{125}$I eCG to the walls of the 3DT tubes. 55,000cpm $^{125}$I eCG in 100ul PBS/Az, 2% FCS, was added to each 3DT tube and incubated for 18 hours at 4°C with constant shaking on a Micro-shaker (Cat. No. AM69, Dynatech, England). 50ul of RAM was then added, mixed and incubated for a further hour at room temperature. 5ul of normal mouse serum was added to increase the volume of precipitate 1 minute prior to precipitate collection by centrifugation (3500g for 30 minutes at 4°C). The volume of normal mouse serum added had previously been established by titration as sufficient to increase the precipitate bulk, and insufficient to saturate the quantity of second antibody used. Supernatants were removed by aspiration and the precipitate washed by resuspension in 2ml PBS/Az and centrifuged as above. The precipitates were then counted using a Packard gamma counter.

The concentration of each monoclonal antibody capable of binding approximately 66% of the maximal precipitable counts was considered suitable for use in the construction of standard curves. The maximum percentage of the total counts precipitated ranged from 32% to 38% of counts added. Higher percentages were not precipitated and reasons for this include damage to the trace during iodination and/or storage, and, heterogeneity of the $^{125}$I labelled eCG.

Duplicate samples of standard assay blanks and negative controls were run for each monoclonal antibody. The eCG standard concentrations ranged from 4.6iu/ml to 10,000iu/ml, and were prepared by dissolving eCG supplied as "Folligon Serum Gonadotrophin" (batch 376551 Intervet, Holland), in PBS/Az containing 2% FCS. 100ul aliquots of each standard were dispensed into 3DT tubes and then 55,000 cpm $^{125}$I eCG in 100ul of PBS/Az containing 2% FCS was added per tube. 0.5ml volumes of each suitably diluted monoclonal antibody were added and incubated with shaking at 4°C overnight. Immune complexes were immunoprecipitated using RAM and washed as described for the construction of the antibody dilution curves.

## EXAMPLE 2

The extraction of purified, biologically active eCG from crude extracts of pregnant mare serum by affinity chromatography confirms the specificity of the antibody and allows accurate assessment of the biological properties of purified eCG.

### (i)>MATERIALS AND METHODS

#### Preparation of affinity columns

Approximately 200mg of each monoclonal antibody was purified from ascites fluid and coupled to Affigel 10 at 16mg per ml of gel. It was established that 89% of monoclonal antibody F50-37, and 94% of F51-99 coupled with the gel during the two reactions. Three columns were prepared for each affinity gel preparation, one of 11ml packed gel volume in a reverse flow column (K 16/20 Pharmacia, NSW) and two of 0.5ml packed gel volumes in 1ml disposable syringes (Terumo, Melbourne). The two small columns were used in establishing optimal conditions for the binding and elution of antigen.

The procedures used for determining elution and binding conditions for each affinity column were the same and consequently the method reported here is only for the monoclonal antibody F50-37 affinity column. The optimal conditons established for the F51-99 affinity column are presented in the results section.

#### Establishment of Elution and Loading Conditions for the F50-37 affinity column:

Iodinated eCG was loaded onto a 0.5ml affinity column in PBS/Az. The column was then washed with the same buffer to remove unbound material from the column. When wash effluents had radioactivity counts of background levels, stepwise elutions of the column with 0.1M citric acid solutions ranging in pH from 6.75 to 2.0 were collected as 2ml fractions.

The procedure was repeated with the second 0.5ml column onto which 100iu eCG (Folligon Serum Gonadotrophin batch 376551, Intervet, Holland) had been loaded in the same buffer. Fractions collected were diluted 1:10 and measured for eCG activity using the MIP assay.

#### Optimisation of eCG binding to the F50-37 affinity column:

Various buffers including phosphate, Tris and citrate-phosphate were examined at 0.5M and 0.1M concentrations over a wide range of pH. Buffer type and molarity had minimal effect on the binding of eCG and the citrate-phosphate buffer was chosen for further investigation. A pH range of 3.5 - 8.0, was obtained by mixing various proportions of 0.1M citric acid and 0.2M $Na_2HPO_4$.

0.5ml samples of each buffer containing approximately 100,000cpm $^{125}$I eCG were prepared in 3DT tubes and 50ul of resuspended F50-37 affinity gel was added to each tube. After overnight incubation at 4°C with shaking, supernatants were removed by aspiration. The gel was then washed twice with 3ml of the appropriate binding buffer, aspirated and counted. Gel samples were then washed with 0.1M citric acid pH 3.0 eluting buffer, and the supernatant collected for counting.

Once optimal binding and elution conditions had been established, a large scale trial was undertaken using the 11ml reverse flow column. A crude eCG·extract binding solution containing 280,000iu eCG was prepared by dissolving the crude freeze-dried eCG sample in 150ml pH 6.5 citrate-phosphate loading buffer and then dialyzed against the same buffer overnight at 4°C.

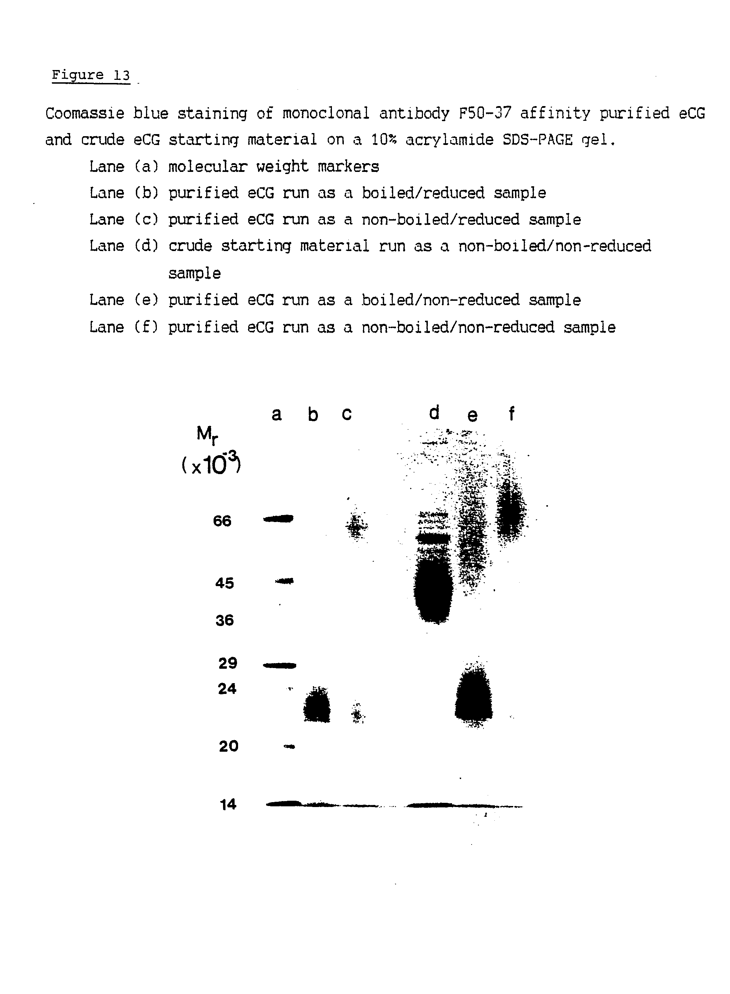

The column was equilibrated with loading buffer and the sample loaded at 5ml/hr at 4°C. Unbound protein was washed from the column with 50ml of loading buffer. The purified product was eluted by reverse flow elution with 0.1M citric acid pH 3.0 at 5ml/hr (see Figure 13).

MIP and rat bioassay analyses of potency :

Samples of each affinity purified product and the crude extract starting material were examined for eCG potency by the MIP assay and rat bioassay.

SDS-PAGE analysis of the affinity purified eCG:

The eluted peak was pooled, dialysed against double distilled water and freeze dried. A 1mg/ml solution of the final product was prepared for SDS-PAGE, MIP assay and rat bioassay analysis of purity and potency. SDS-PAGE analysis of the product included Coomassie blue staining of gels, and, amino black and immunostaining of Western transferred proteins, run as non-reduced/non-boiled, non-reduced/boiled and reduced/boiled samples.

(ii) RESULTS:

Optimization of eCG loading and elution of the F50-37 affinity column:

The results for the elution of $^{125}$I eCG and MIP assay positive eCG are shown in Table 1. Results for both experiments coincide in that pH 3.0 0.1M citric acid elutes the bulk of eCG bound whether it is iodinated or non-iodinated from F50-37 affinity column gel.

21

Table 1

Effect of varying pH on the elution of iodinated eCG and eCG detected using the MIP assay from 0.5ml F50-37 affinity columns using a 0.1M citric acid pH gradient as presented.

| Elution Buffer | CPM $^{125}$I eCG in Sequential Fractions | MIP Assay Detectable eCG |
|---|---|---|
| PBS Wash | 2,940 | − |
| PBS Wash | 2,380 | − |
| 0.1M Citric Acid | | |
| − pH 6.75 | 2,040 | − |
| − pH 6.0 | 3,930 | − |
| − pH 5.5 | 8,260 | − |
| − pH 5.0 | 11,710 | − |
| − pH 4.5 | 25,050 | − |
| − pH 4.0 | 53,620 | + |
| − pH 3.5 | 70,870 | + |
| − pH 3.0 | 59,450 | + |
| − pH 2.5 | 11,220 | − |
| − pH 2.0 | 4,510 | − |
| PBS Wash | 5,770 | |

Optimal complexing of eCG to the F50-37 affinity column was obtained at pH 6.0 and above as shown in Table 2.

22

Table 2

The effect of varying pH on the binding of $^{125}$I eCG to F50-37 affinity column gel.

| pH of Loading Buffer | Total Counts Bound | Counts Eluted with pH 3.0 0.1M Citric Acid |
|---|---|---|
| 3.5 | 20,935 | 5,910 |
| 4.0 | 23,080 | 8,120 |
| 4.5 | 29,515 | 15,190 |
| 5.0 | 32,030 | 19,095 |
| 5.5 | 34,355 | 20,715 |
| 6.0 | 48,915 | 28,295 |
| 6.5 | 42,760 | 29,540 |
| 6.75 | 41,760 | 26,785 |
| 7.0 | 43,800 | 24,725 |
| 7.5 | 45,445 | 28,390 |
| 8.0 | 47,060 | 24,460 |

The optimum loading and eluting buffers for the F50-37 monoclonal antibody affinity column were, 0.1M citric acid - 0.2M Na$_2$HPO$_4$ pH 6.5, and 0.1M citric acid pH 3.0 respectively.

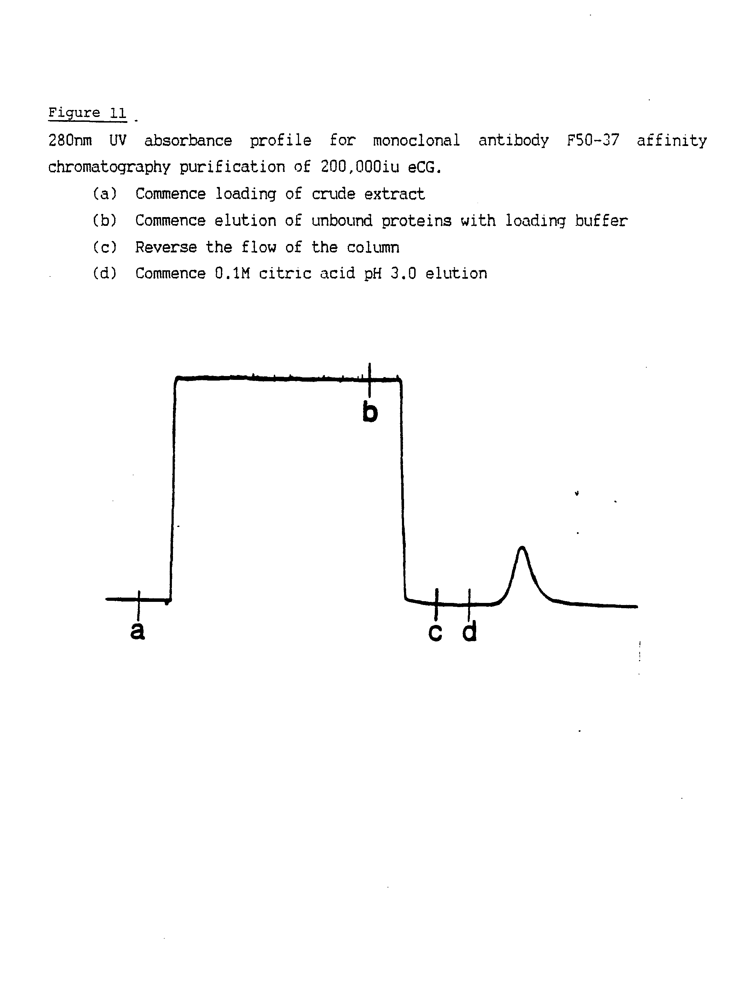

The buffers suitable for the elution and binding of eCG for the F51-99 monoclonal antibody affinity column were, binding in PBS/Az and elution with 0.1M NaCl, 0.1M glycine adjused to pH 2.0 with 1M HCl. The elution of the purified eCG from this column required conditions considerably more harsh than those required for elution from the F50-37 monoclonal antibody affinity column. Consequently, the eCG purified on the F50-37 column was chosen for use in Western transblotting experiments. Sufficient monoclonal antibody F51-99 affinity purified eCG was prepared for SDS-PAGE analysis and comparison with the eCG purified on the F50-37 affinity column (Figure 11), and, MIP and rat bioassay analyses of potency.

Monoclonal antibody F50-37 affinity chromatography purification of eCG:

Of the 280,000 iu eCG loaded onto the column, 80,000iu failed to bind during the single passage through the column, and were subsequently detected by MIP assay in the loading and wash fractions. The same procedure was repeated using the F51-99 affinity column.

MIP and rat bioassay analysis of potency:

The specific activities of (i) the crude extract, and (ii) both of the affinity column final purified products were 660iu/mg and 11,250 iu/mg respectively as measured by MIP assay. Bioassay results showed both of the final products had potencies 10,000iu/mg.

23

Examination of purified eCG:

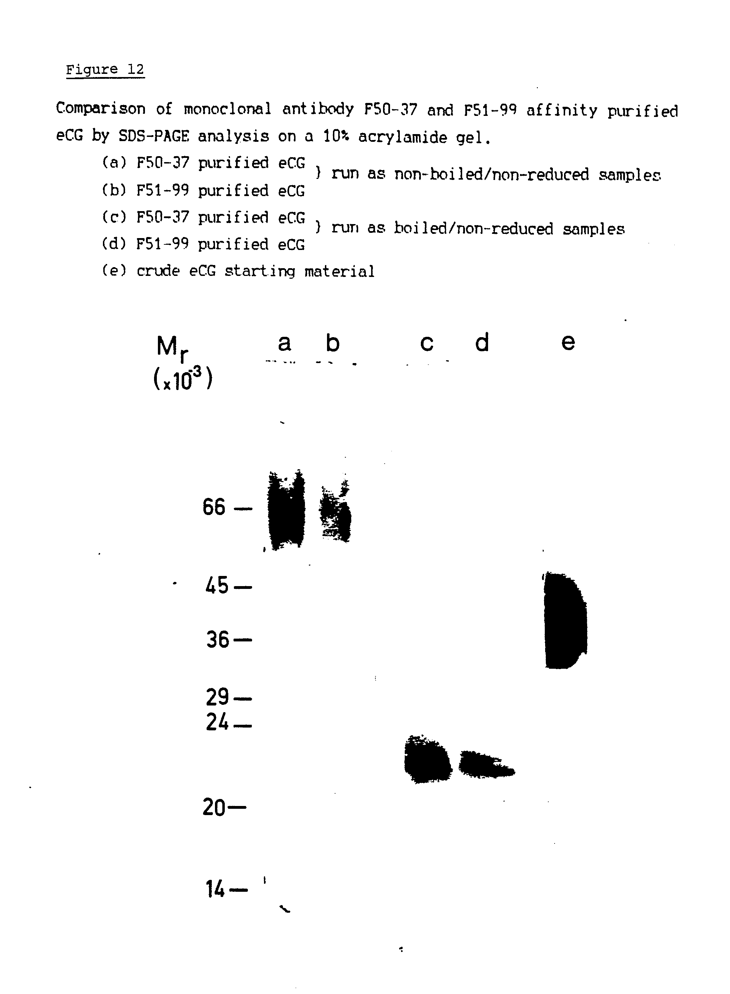

Affinity purified eCG preparations from the monoclonal antibody F50-37 and F51-99 affinity columns were indistinguishable by SDS-PAGE analysis (Figure 12) and, MIP, ELISA and rat bioassay analyses of potency Coomassie blue staining of non-reduced/non-boiled samples run on SDS-PAGE showed one diffuse band around $M_r$ 66,000. This band was not present in the crude extracts run on SDS-PAGE (Figure 13). The same band was immunoreactive on nitrocellulose following Western transfer with the three eCG monoclonal antibodies and showed no reactivity with the negative control monoclonal antibody (Figure 14).

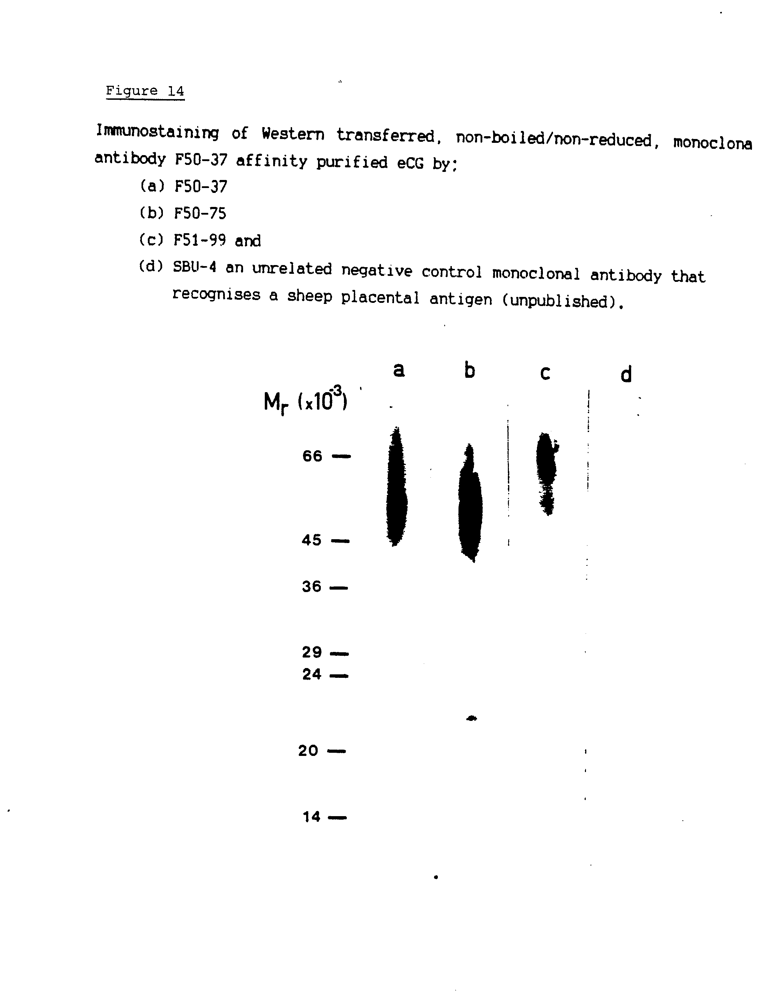

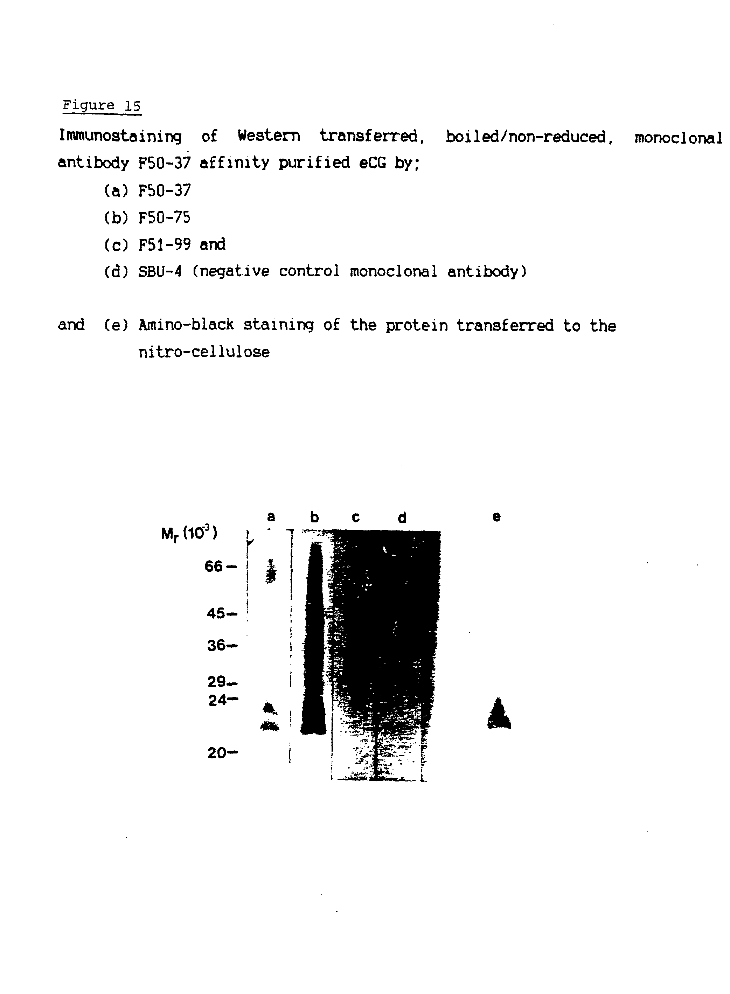

Boiling in non-reducing buffer dissociated the $M_r$ 66,000 band to give a very diffuse $M_r$ 55,000 band and a diffuse $M_r$ 22,000 band with three associated discrete bands (Figure 13, Lane e). Immunostaining of Western transferred boiled/non-reduced samples differs for each monoclonal antibody (Figure 14). Monoclonal antibody F51-99 was unreactive with all subunits and fragments while F50-37 and F50-75 stained heavily in the $M_r$ 22,000 region. Monoclonal antibody F50-75 also gave strong staining in the $M_r$ 36,000 region. Boiling samples for five minutes in reducing buffer gave similar Coomassie blue staining on SDS-PAGE gels to boiling in non-reducing buffer (Figure 13). All monoclonal antibodies were unreactive with reduced samples.

Monoclonal antibody affinity chromatography purification of immunologically and biologically active eCG using the monoclonal antibodies F50-37 and F51-99 has confirmed their reactivity with eCG. Immunological reactivity of the purified eCG as shown by Western transfer and immunostaining with the remaining eCG monoclonal antibody, F50-75, also confirmed its reactivity with eCG.

The high purity of the affinity purified eCG products has been shown by, SDS-PAGE Coomassie blue staining of a single diffuse band of approximately $M_r$ 66,000, and by the high biological activity of the purified eCG in both MIP assay and rat bioassay. SDS-PAGE results, and MIP assay and bioassay estimations of the specific activity for each affinity purified eCG preparation are consistent with those previously reported for purified eCG [Gospodarowicz, Endocrinology, 91, 101-106, (1971)]; Moore and Ward, (1980)]. The broadness of the bands appearing in the SDS-PAGE gels following Coomassie blue staining, is also consistent with previous findings [Moore and Ward, (1980)].

## Monoclonal Antibody F51-99 Affinity Purification of eCG from Plasma

An initial experiment was undertaken to examine the effect of temperature of Affigel F51-99 gel extraction of eCG directly from plasma. Three temperatures were compared: 4°C, 20°C and 37°C.

750ml pregnant mare plasma +0.1% azide and thiomersol was filtered using several Whatman No1 filter papers. 3 aliquots of 250ml plasma, each containing 3.5ml Affigel F51-99 gel (packed gel volume), were then rotated overnight at the different temperatures stated.

After settling for 1 hour the majority of the plasma was syphoned off. The residual plasma was then swirled to resuspend the gel suspensions and a column then packed using each entire volume.

The three Affigel F51-99 columns were then washed extensively with PBS/Az to remove residual plasma proteins. Each column was eluted using 0.1M NaCl, 0.1M Glycine pH 2.0 buffer and the eCG peak pooled. This solution was immediately buffered to pH 6.5 to avoid possible damage to the eCG due to prolonged exposure to this extreme pH.

The 3 separate eCG products were dialysed and assayed for comparison.

Results showed Affigel F51-99 gel absorptions of eCG from plasma serum to be as effective at 4°C as at 37°C and/or 20°C.

A large scale trial was then undertaken at 4°C incorporating 11 ml of Affigel F51-99 and 2.5 litre of pregnant mare plasma containing 49 iu/ml eCG. After overnight rotational mixing, the gel was removed, washed and eluted as above. The eCG product was assayed and showed a recovery of 120-140,000 iu total, effectively 100%. The plasma remaining after extraction was negative in all eCG assays. The eCG product was biologically active and of very high purity (8,000 iu/mg). This specific activity was increased to 12,500 iu/mg following a second Affigel F51-99 chromatography step which removed the contaminating fibrinogen.

## EXAMPLE 3

Large scale affinity purification of eCG from serum and crude serum extracts using AFFIPREP 10 immobilized monoclonal antibody F51-99

Affiprep 10 is a more stable matrix for immobilising proteins such as monoclonal antibodies for the production of affinity column gels. Approximately 200 mg of F51-99 was coupled to 20 mls of Affiprep 10 as described by the manufacturer. This affinity matrix (Affiprep 10/51-99) was then examined in 2 eCG purification trials.

Trial 1

A crude extract (325 iu/mg) eCG preparation (500,000 iu) was dissolved in 200 ml PBS/Az and mixed in a roller bottle overnight at 4°C. The Affiprep 10/51-99 gel was collected into an affinity column, washed and eluted with 0.1M NaCl, 0.1M glycine pH 2.0.

The eluted peak was quickly buffered to pH 6.0, dialysed against distilled water and freeze dried.

ELISA and rat bioassay analyses of specific activity were consistent and gave activities of 11,600 and > 10,000 iu 1 mg respectively. 420,000 iu was recovered in the purified product and 60,000 iu were detected in the residual binding solution.

Trial 2

The Affiprep 10/51-99 gel was reacted overnight with 2 litres of pregnant mare plasma containing 68 iu/ml as described in trial 1. 125-130,000 iu were recovered with a specific of approximately 5-6,000 iu/mg. This was increased to 10,000 iu/mg by a second column chromatography purification of the eCG using the same Affiprep 10/51-99 affinity gel.

EXAMPLE 4

Another trial was undertaken in sheep to compare the time of eCG administration, and the effect of antibody vs no antibody administration.

Three doses of affinity purified (Affiprep 10/51-99) eCG were given at either (i) time of progesterone plug pull or (ii) 48 hours before plug pull (-48 hrs). Half of the sheep in each group received 1 mg of monoclonal antibody at 48 hours after plug pull while the other half received an injection of saline only.

## Results

| Time of eCG | Dose | Antibody +/- | No of C.L. | No of Follicles |
|-------------|------|--------------|------------|-----------------|
|             | "0"  | -            | 1.64       | -               |
|             | 800  | -            | 7.8        | 0.4             |
|             | 800  | +            | 7.3        | 0.0             |
| - 48 Hrs    | 1200 | -            | 10.6       | 1.7             |
|             | 1200 | +            | 10.8       | 3.0             |
|             | 1600 | -            | 9.5        | 1.7             |
|             | 1600 | +            | 10.0       | 1.0             |
|             | 800  | -            | 3.3        | 1.0             |
|             | 800  | +            | 4.2        | 0               |
| 0 Hrs       | 1200 | -            | 3.4        | 1.4             |
|             | 1200 | +            | 6.0        | 0.75            |
|             | 1600 | -            | 4.2        | 4.2             |
|             | 1600 | +            | 6.8        | 1.6             |

The number of corpora lutea obtained at -48 hours was significantly higher for all groups than those receiving eCG at plug pull.

The numbers of unwanted large follicles in all groups were again low when compared with previous trials incorporating crude commercial eCG preparations. These were reduced even further by the administration of a single antibody dose at +48 hours.

The reduction in the number of large follicles in antibody treated ewes is also accompanied by a rise in the numbers of corpora lutea and therefor ovulations.

The use of the monoclonal antibody has increased the ovulation rate and reduced the number of undesirable large follicles.

## EXAMPLE 5

Five doses of a commercially available eCG were compared with five equivalent doses of this material after purification of the Affiprep 10/51-99 affinity column.

## Results

| Dose eCG | Number of Corpora Lutea | | Number of Large Follicles | |
|----------|------|------|------|------|
|          | Crude | Pure | Crude | Pure |
| "0"      | 1.43 |      | 0.18 |      |
| 200 iu   | 1.75 | 1.55 | 0.25 | 0.75 |
| 400 iu   | 2.66 | 2.34 | 0.12 | 0.06 |
| 800 iu   | 4.38 | 2.95 | 1.00 | 0.27 |
| 1200 iu  | 6.76 | 6.25 | 3.37 | 1.75 |
| 1600 iu  | 6.61 | 7.75 | 8.45 | 2.69 |

26

The results show a greatly reduced number of large follicles in those ewes receiving the purified eCG in the higher dose range.

EXAMPLE 6

A trial was undertaken in pigs to examine whether purified eCG alone or purified eCG plus antibody F51-99 could improve litter size. Three groups of sows were compared. Each group received 2 injections, one at time of weaning and one at the time of first mating.

## Results

| No. of Litters | 1st Inject. | 2nd Inject. | Av. Litter Size | % Still Born | Av. Born Alive Litter Size |
|---|---|---|---|---|---|
| 72 | Saline | Saline | 10.11 | 6.2% | 9.49 |
| 74 | 1500iu eCG | Saline | 10.91 | 7.1% | 10.14 |
| 68 | 1500iu eCG | 1mg 51-99 | 10.87 | 5.8% | 10.24 |

These sows receiving both eCG and antibody on average gave birth to an extra 0.75 live piglets per litter.

EXAMPLE 7

This example examines the ability of the monoclonal antibodies F50-37, F50-75 and F51-99 to block and/or modify the effect of 1600iu eCG in the ewe. Ideally an eCG superovulation protocol incorporating the use of one or more of the eCG monoclonal antibodies would result in high numbers of ovulations with reduced numbers of post-ovulatory cystic follicles.

MATERIALS AND METHODS

Oestrus in Merino/Border Leicester cross ewes was synchronized by intravaginal insertion of progesterone sponges (Repromap 60mg MAP, Upjohn, Sydney) for 13 days. Immediately prior to eCG injection of the ewes, the sponges were removed and the sheep given one of the following treatments.

Trial 1

Treatment

    (a) Intramuscular (IM) injection of saline
    (b) IM injection of 1600iu eCG (Pregnicol, Heriot Agencies, Melb.) in 1ml saline
    (c) IM eCG as in (b) plus immediate IV (intravenous) injection of
        (i) 1mg antibody F50-37 or
        (ii) 1mg antibody F50-75 or
        (iii) 1mg antibody F51-99, all in 1ml of saline.

Trial 2

Treatment

   (a) IM injection of saline

   (b) IM injection of 1600iu eCG (as for Trial 1)

   (c) IM injection 1600iu eCG at plug pull (as in Trial 1) plus, 49 hours after eCG injection, an IM injection of

      (i) 1mg antibody F50-75 or

      (ii) 1mg antibody F51-99, each in 1ml of saline.

Ovarian response was assessed by mid-ventral laproscopy 7-8 days after oestrus. The number of corpora lutea and follicles were recorded.

In Trial 1, a significant block of eCG activity was found in the eCG treated sheep following the injection of monoclonal antibody F51-99 compared with sheep receiving eCG only (Table 3). The probability that the mean responses of eCG only, and eCG/F51-99 treated sheep were not different was .0.001 using the unpaired t-test (Statsworks program, Model 512KA computer, Macintosh Apple). The mean number of ovulations, represented by the mean number of corpa lutea (CL) was lower in sheep receiving both eCG and F51-99 than in sheep receiving saline only. No follicles were found with either treatment. In sheep receiving eCG only, eCG plus monoclonal antibody F50-37, and, eCG plus monoclonal antibody F50-75, typical superovulatory responses were observed. Monoclonal antibody F50-75 may have partially inhibited the eCG superovulatory action but this did not appear significant.

Trial 2 results were consistent with Trial 1 (Table 3). Intramuscular injection of monoclonal antibody F50-75 48 hours after eCG injection failed to decrease the superovulatory action of eCG. Sheep injected with monoclonal antibody F51-99, 48 hours after eCG injection, had considerably fewer follicles and a slightly reduced number of ovulations when compared to eCG only treated sheep. The difference in ovulation rates were not significant. Monoclonal antibody F50-37 was excluded from the second trial due to the lack of activity shown in Trial 1 results.

Table 3

Summary of the results for Trials 1 and 2 on the modification of eCG bioactivity in the ewe by monoclonal antibodies to eCG.

| Dose of eCG | Time of antibody Treatment | Number of Ewes | Mean Response CL | Follicles |
|---|---|---|---|---|
| **Trial 1** | | | | |
| 0 | – | 22 | 1.95 | 0 |
| 1600iu | – | 6 | 6.66 | 1.16 |
| 1600iu | Immediate IV F50-37 | 5 | 5.4 | 1.2 |
| 1600iu | Immediate IV F50-75 | 5 | 4.4 | 0.6 |
| 1600iu | Immediate IV F51-99 | 5 | 1.4 | 0 |
| **Trial 2** | | | | |
| 0 | – | 7 | 1.14 | 0 |
| 1600iu | – | 8 | 6.38 | 2.62 |
| 1600iu | 48 hrs IM F50-75 | 7 | 6.57 | 3.43 |
| 1600iu | 48 hrs IM F51-99 | 7 | 4.43 | 0.43 |

CL = Corpora Lutea

28

The results of both trials showed F51-99 to be an effective monoclonal antibody for controlling eCG bioactivity in the ewe. Trial 1 results showed that 1mg monoclonal antibody F51-99 when injected IV immediately after eCG administration, totally abolished the superovulatory activity of 1600iu eCG in the ewe. Ewes receiving both eCG and monoclonal antibody F51-99, had fewer ovulations than those receiving no eCG at all. Monoclonal antibodies F50-37 and F50-75 had no effect under the same conditions.

Differences in the results of the two trials shows that variation in time and route of monoclonal antibody administration can affect the results obtained. A significant difference is seen between eCG/F51-99 treated ewes in Trials 1 and 2. The probability that the means of each group were not different was $Pr < 0.05$. By delaying the administration of the antibody in Trial 2, a substantial proportion of the desired eCG superovulatory activity is obtained with only minimal ovarian overstimulation. The mean number of unruptured follicles in this group was 0.43, while those receiving no antibody or monoclonal antibody F50-75 had mean numbers of 2.62 and 3.43 follicles per ewe respectively. This difference was not statistically significant due to the small numbers of sheep used, and the distribution of data within each group. The decreased number of ovulations associated with the decrease in unruptured follicle numbers in F51-99 treated sheep, was an undesirable effect but was not statistically significant. It is likely however, that with better timing and optimisation of the amount of the antibody administered, the desired reduction in numbers of unruptured follicles may be obtained without a decrease in the number of ovulations.

Coinciding with decreased numbers of unruptured follicles should be an expected decrease in oestrogen levels. A similar return to physiological oestrogen levels in sheep, may permit natural mating for embryo transfer programs. To date natural matings of superovulated sheep have produced poor yields of embryos, which is probably the result of decreased sperm mobility/penetration into the uterus [Killeen, Proc. Symp Embryo Transfer in Cattle, Sheep and Goats. Published ASRB (1982)]. As non-surgical artificial insemination is extremely difficult in sheep due to the constriction of the cervix, surgical or laproscopic insemination of the uterine horns has been adopted with good results [Trounson and Moore, Aust. J. Biol. Sci., 27, 301-309, (1974)]. Studies have commenced to examine the possibility of natural mating following superovulation with eCG when controlled by the monoclonal antibody, F51-99.

Trial Three

A further trial was conducted to analyse the effect of antibody dose rate on ovarian response. The results are set out in Table 4. Sheep received 1600 iu eCG at sponge withdrawal and the various antibody doses were administered 48 hours later.

TABLE 4

Sheep Trial

| n=12 (No. of ewes per group | mean CL (corpus lutea) | mean follicles |
|---|---|---|
| High dose   3mg | 5.9 | 3.1 |
| Mid dose   0.75mg | 6.2 | 1.0 |
| Low dose   0.25mg | 5.8 | 2.6 |
| No Dose "0" antibody CONTROL | 6.0 | 3.45 |

The results show the need for optimization of antibody dosage.

EXAMPLE 8

BOVINE DATA

Trials have been undertaken in cows superovulated with eCG. The use of the monoclonal antibody F51-99, 72 and 96 hours after eCG injection has demonstrated that the benefits resulting from the use of this monoclonal antibody usage in the ewe are also applicable in cattle.

## Cattle Trial Results

|  |  | eCG only | |
|---|---|---|---|
| Overall | Ovulation rate | Follicles |
| means | 14.25 | 3.0 |
|  | n = 12 | |

eCG & F51-99 (72 hrs)

| | Ovulation rate | Follicles |
|---|---|---|
| Overall | 4.5 | 1.8 |
| means | n = 12 | |

eCG & F51-99 (96hrs)

| | Ovulation rate | Follicles |
|---|---|---|
| Overall | 10.1 | 1.7 |
| means | n = 11 | |

EXAMPLE 9

TWINNING DATA

Depending on the batch of eCG used, 200-800 iu eCG injected at the time of sponge removal and a subsequent injection of 0.1 - 0.5 mg of antibody will induce a higher number of twin births than in non-eCG treated or eCG-non-antibody treated sheep. eCG may also be administered prior to sponge removal. The important fact irrespective of the regime chosen to induce double ovulation is that low doses of PMSG and a subsequent dose of antibody can effectively increase the incidence of twin births in sheep and other live stock including cattle and goats.

EWE DATA

SPONGE 13 days. 800 iu at time of sponge removal.
48 hours later either (i) saline injection (1 ml)
           (ii) 1ml 0.5 mg/ml F51-99 in Sal.

|  | Quads | Triplets | Twins | Single | None |
|---|---|---|---|---|---|
| eCG only | 1 | 4 | 1 | 0 | 3 |
| eCG & F51-99 | 0 | 1 | 4 | 4 | 1 |

EXAMPLE 10

Trials have been undertaken in cows superovulated with pure eCG purified by monoclonal antibody affinity chromatography using the monoclonal antibody F51-99. The use of different doses demonstrated that pure eCG can be used at much lower concentrations to achieve superovulation compared to commercially available preparations. The use of low doses of eCG resulted in no overstimulation of ovaries, a serious problem with the presently available commercial preparations of eCG used for superovulation.

**Hereford Cattle. 3-4 years of age, 2nd or 3rd calving.**
**Average Liveweight 344-371 Kg (range)**

| Treatment | No. Treated | Ovul'n Rate(Mean) | Range | O.I.** (Mean) | Comments on Ovary Appearance |
|---|---|---|---|---|---|
| A 6000 iu pure | 8 | 1.8 | 1-3 | 4.0 | All signif. overstimulated |
| B 3000 iu pure | 8 | 6.0 | 1-16 | 3.7 | All signif. overstimulated |
| C 1500 iu pure | 8 | 7.8 | 2-14 | 2.0 | 5 - Normal 3 - Signif. over-stimulation |
| D 750 iu pure | 8 | 8.0 | 1-18 | 0.7 | 6 - Normal 2 - Slight over-stimulation |
| E 3000 Tolligon* | 8 | 9.0 | 1-19 | 1.9 | 6 - Signif. over-stimulation 2 - Normal |
| F Saline control | 8 | 1.0 | 1-1 | 0.3 | 6 - Normal 2 - Slight over-stimulation |

* Tolligon - commercially available preparation (Intervet, Australia)

** O.I. refers to Overstimulation index "0" - normal, "5" overies as large as tennis balls

Comments

Use of pure eCG 750 iu gives same superovulation response as 3000 iu of the commercially available preparation Folligon. No overstimulation of ovaries observed with pure eCG at 750 iu. Use of 3000 iu of Folligon produced significant overstimulation.

It is therefore apparent that much lower amounts of eCG may be used when purified according to the process of the present invention to achieve same number of ovulations with a near complete absence of overstimulation compared to commercially available products.

Finally, it is to be understood that various other modifications and/or alterations may be made without departing from the spirit of the present invention as outlined herein.

## Claims

1. A process for producing a monoclonal antibody against equine chorionic gonadotrophin (eCG) which method includes

    (a) providing a B cell capable of producing antibodies against equine chorionic gonadotrophin, and a myeloma cell,

    (b) fusing the B cell with the myeloma cell,

    (c) propagating a hybridoma formed thereby, and

    (d) harvesting an antibody produced by said hybridoma.

2. A process according to claim 1 wherein
the B cell capable of producing antibodies is obtained from an animal immunised with equine chorionic gonadotrophin or an immunogenic fragment thereof; and
the myeloma cell line is an NS-1 type myeloma cell line.

3. A process according to claim 2 wherein the fusion step includes mixing somatic cells with myeloma cells in the presence of a fusion promoting agent selected from Sendai virus and polyethylene glycol.

4. A process according to claim 3 wherein the hybridoma propagation step includes
detecting antibody producing hybridomas utilising an assay selected from enzyme linked immunoassay, radioimmunoassay or immunohistology;
subsequently cloning the fused cell hybrid selected into individual antibody producing cell lines; and
propagating each cell line in-vivo or in-vitro.

5. A continuous cell line which produces a monoclonal antibody against equine chorionic gonadogrophin, including a hybridoma formed by fusing a B cell capable of producing antibodies against equine chorionic gonadotrophin with a myeloma cell.

6. A continuous cell line according to claim 5 formed by fusing an NS-1 type myeloma cell with a spleen B cell obtained from a BALB/c mouse immunised with equine chorionic gonadotrophin.

7. A continuous cell line selected from F50-37 F50-75, F51-99, F65-88, F65-98, F65-191 and F65-130 as hereinbefore described.

8. A monoclonal antibody against equine chorionic gonadotrophin produced from a continuous cell line which produces a monoclonal antibody against equine chorionic gonadogrophin, including a hybridoma formed by fusing a B cell capable of producing antibodies against equine chorionic gonadotrophin with a myeloma cell.

9. A monoclonal antibody formed by the continuous cell line selected from F50-37, F50-75, F51-99, F65-88, F65-98, F65-191, and F65-130.

10. A method for the detection of equine chorionic gonadotrophin (eCG) in an equine animal, which method includes providing
a sample from the equine animal to be tested; and
a monoclonal antibody against equine chorionic gonadotrophin produced from a hybridoma cell line, or mixtures thereof;
contacting the monoclonal antibody(s) with a portion of the sample to bind any equine chorionic gonadotrophin present; and
subjecting the bound sample to a detection assay.

11. A method according to claim 10 including
providing an IgM class eCG monoclonal antibody or mixtures thereof and a source of equine chorionic gonadotrophin (eCG) coated mammalian blood cells;
contacting the monoclonal antibody(s) with a portion of the sample to bind any chorionic gonadotrophin present; and
contacting the mixture of antibody and sample with the eCG coated mammalian red blood cells and subjecting the product thereof to a visual inspection.

12. A method according to claim 10 including

providing an IgG class eCG monoclonal antibody or mixtures thereof conjugated to a suitable enzyme;

contacting the enzyme conjugated monoclonal antibody(s) with a portion of the sample to bind any equine chorionic gonadotrophine present; and

subjecting the product formed thereof to an ELISA detection.

13. A test kit including

a source of monoclonal antibody against equine chorionic gonadotrophin or mixtures thereof in a buffered solution in a suitable container,

a source of equine chorionic gonadotrophin-coated mammalian red blood cells in a suitable container; and

a solid support.

14. A test kit according to claim 13 wherein the solid support is microtitre plate or tray.

15. A test kit including

a source of an enzyme conjugated monoclonal antibody or mixtures thereof in a suitable container;

a solid support including an amount of a further monoclonal antibody or mixtures of monoclonal antibodies against equine chorionic gonadotrophin bound thereto.

16. A test kit according to claim 15 wherein the conjugated monoclonal antibody is a urease conjugated monoclonal antibody formed by hybridoma cell line F51-99 and the bound monoclonal antibody is selected from monoclonal antibodies formed by hybridoma cell lines F50-37 or F50-75.

17. A method of purifying equine chorionic gonadotrophin (eCG) which method includes

providing a source of impure eCG and a monoclonal antibody against equine chorionic gonadotrophin formed by a hybridoma cell line;

passing the impure eCG through the monoclonal antibody matrix to capture the eCG;

and eluting the captured eCG from the monoclonal antibody matrix.

18. A method according to claim 17 wherein the matrix is an affinity chromatographic gel.

19. A method according to claim 18 wherein the antibody matrix support is in a chromatography column or in a large scale container or vat which may be rotated, agitated or stirred.

20. A method according to claim 19 wherein the monoclonal antibody is selected from monoclonal antibodies formed by hybridoma cell lines F50-37 and F51-99.

21. A method according to claim 20 wherein the impure equine chorionic gonadotrophin source is buffered solution of pregnant mare plasma or sera at a pH of approximately 6.0 to 8.0,

22. A method according to claim 21 wherein the elution step is conducted utilising a sodium chloride-glycine solution buffered to a pH of approximately 2.0 to 3.0.

23. A purified equine chorionic gonadotrophin (eCG) characterised in that in use there is a reduced incidence of large follicles in the ovaries of the animal treated.

24. A method of inducing oestrus and/or increasing fecundity in female animals which method includes administering to the animal an effective amount of a purified equine chorionic gonadotrophin characterised in that in use there is a reduced incidence of large follicles in the ovaries of the animal treated.

25. A method according to claim 24 wherein the animal to be treated is a sheep, goat or cow and the amount of equine chorionic gonadotrophin administered is in the range of approximately 250 to 3000 iu.

26. A method according to claim 25 further including administering simultaneously with or after equine chorionic gonadotrophin administration to the animal approximately 0.25 to 1.0 mg of a purified monoclonal antibody against equine chorionic gonadotrophin formed by a hybridoma cell line.

27. A method according to claim 24 wherein the animal to be treated is a pig and the amount of equine chorionic gonadotrophin administered is in the range of approximately 500 to 1500 iu.

28. A method according to claim 27 further including administering simultaneously with or after equine chorionic gonadotrophin administration to the animal approximately 0.5 to 1.5 mg of a purified monoclonal antibody against equine chorionic gonadotrophin formed by a hybridoma cell line.

29. A method of substantially increasing the number of ova produced during a single oestrus cycle in female animals which method includes administering to the animal an effective amount of a purified equine chorionic gonadotrophin characterised in that inuse there is a reduced incidence of large follicles in the ovaries of the animal treated.

30. A method according to claim 29 wherein the animal to be treated is a sheep or goat and the amount of equine chorionic gonadotrophin administered is in the range of approximately 500 to 2000 iu.

31. A method according to claim 30 further including administering simultaneously with or after equine chorionic gonadotrophin administration to the animal approximately 0.50 to 2.0 mg of a purified monoclonal antibody against equine chorionic gonadotrophin formed by a hybridoma cell line.

32. A method according to claim 29 wherein the animal to be treated is a cow and the amount of equine chorionic gonadotrophin administered is in the range of approximately 500 to 3000 iu.

33. A method according to claim 32 further including administering simultaneously with or after equine chorionic gonadotrophin administration to the animal approximately 0.5 to 3 mg of a purified monoclonal antibody against equine chorionic gonadotrophin formed by a hybridoma cell line.

34. A method according to claim 29 wherein the animal to be treated is a pig and the amount of equine chorionic gonadotrophin administered is in the range of approximately 500 to 1500 iu.

35. A method according to claim 34 further including administering simultaneously or after equine chorionic gonadotrophin administration to the animal approximately 0.5 to 1.5 mg of a purified monoclonal antibody against equine chorionic gonadotrophin formed by a hybridoma cell line.

36. A method of increasing ovulation to provide twinning in female animals which method includes administering to the animal an effective amount of a purified equine chorionic gonadotrophin characterised in that in use there is a reduced incidence of large follicles in the ovaries of the animal treated.

37. A method according to claim 36 wherein the animal to be treated is a sheep, goat or cow and the amount of equine chorionic gonadotrophin administered is in the range of approximately 100 to 1000 iu.

38. A method according to claim 37 further including administering simultaneously with or after equine chorionic gonadotrophin administration to the animal approximately 0.25 to 1.0 mg of a purified monoclonal antibody against equine chorionic gonadotrophin formed by a hybridoma cell line.

39. A veterinary composition including
an effective amount of a monoclonal antibody against equine chorionic gonadotrophin formed by a hybridoma cell line,
and a veterinarily acceptable diluent carrier or excipient therefor.

40. A veterinary composition according to claim 39 further including the effective amount of a purified equine chorionic gonadotrophin.

41. A veterinary composition according to claim 40 wherein the composition is a buffered saline solution.

42. A method of inducing abortion in pregnant mammals which method includes administering to the mammal an effective amount of a veterinary composition including
an effective amount of a monoclonal antibody against equine chorionic gonadotrophin formed by a hybridoma cell line,
and a veterinarily acceptable diluent carrier or excipient therefor.

## Figure 1

Immunoprecipitation of iodinated eCG by three putative monoclonal antibodies to eCG. Each putative eCG monoclonal antibody binds to and precipitates iodinated eCG in the presence of the second antibody, RAM. The dilution curves for monoclonal antibodies F50-37 and F51-99 show a prozone effect when neat or at a dilution of 1:1, while monoclonal antibody F50-75 shows no prozone effect. The control monoclonal antibody SBU-4 is negative throughout.

IMMUNOPRECIPITATION OF IODINATED eCG BY THREE PUTATIVE
MONOCLONAL ANTIBODIES TO eCG

Figure  2

ANTIBODY DILUTION CURVES FOR THREE MONOCLONAL ANTIBODIES TO
eC8 USING DOUBLE ANTIBODY PRECIPITATION

The antibody dilution curves for monoclonal antibodies F50-37 and F51-99
show a prozone effect at higher concentrations. Similar counts are
precipitated by each antibody but monoclonal antibody F50-75 requires a
higher concentration of supernatant to accomplish this. The control
monoclonal antibody is negative throughout.

Figure 3

STANDARD CURVE FOR MONOCLONAL ANTIBODY F50-37

Figure 4

STANDARD CURVE FOR MONOCLONAL ANTIBODY F50-75

Figure 5

STANDARD CURVE FOR MONOCLONAL ANTIBODY F51-99

Figure   6

**SCATCHARD ANALYSIS OF THREE MONOCLONAL ANTIBODIES TO eCG**

The affinity of each antibody is represented by the slope of the line. F50-37 with the steepest slope has the highest affinity. The x-intercept of each line gives the concentration of antibody binding sites. F50-37 with the highest affinity requires the least antibody to precipitate a given number of counts and this is reflected in the lower binding site concentration of the antibody.

Figure 7

Positive staining of the large decidual-like binuclear cells (BN) of the pregnant mare (day 60) endometrial cup by three monoclonal antibodies to eCG using HRP labelled RAM. BV = Blood Vessel, EG = Endometrial Gland, G = possible Golgi Apparatus, N = Nuclei (pale staining). x750

    (a)   F50-37

    (b)   F50-75

    (c)   F51-99

Figure 8

Staining of equine (E) and human (H) pituitaries by three monoclonal antibodies to eCG using HRP labelled RAM. x150

(a) F50-37  shows binding to equine pituitary sections only

(b) F50-75  shows binding to both equine and human pituitary sections

(c) F51-99  shows no binding to either equine or human pituitary sections

E                                    H

Figure    9

Staining of ovine pituitary sections by three monoclonal antibodies to eCG
using HRP labelled RAM. x150

    (a)  F50-37  shows no staining

    (b)  F50-75  shows positive staining

    (c)  F51-99  shows no staining

a

b

c

Figure 10

Screening of immune and normal mouse sera by immunohistology on pregnant mare (day 60) endometrial cup sections using HRP labelled RAM. x160

    (a) normal mouse serum diluted 1:100 in blotto

    (b) eCG immune mouse serum diluted 1:100 in blotto, arrows indicate the large positive staining cells of the endometrial cup.

a

b

Figure 11 .

280nm UV absorbance profile for monoclonal antibody F50-37 affinity chromatography purification of 200,000iu eCG.

    (a)   Commence loading of crude extract

    (b)   Commence elution of unbound proteins with loading buffer

    (c)   Reverse the flow of the column

    (d)   Commence 0.1M citric acid pH 3.0 elution

## Figure 12

Comparison of monoclonal antibody F50-37 and F51-99 affinity purified eCG by SDS-PAGE analysis on a 10% acrylamide gel.

(a) F50-37 purified eCG } run as non-boiled/non-reduced samples
(b) F51-99 purified eCG

(c) F50-37 purified eCG } run as boiled/non-reduced samples
(d) F51-99 purified eCG

(e) crude eCG starting material

Figure 13

Coomassie blue staining of monoclonal antibody F50-37 affinity purified eCG
and crude eCG starting material on a 10% acrylamide SDS-PAGE gel.

    Lane (a) molecular weight markers
    Lane (b) purified eCG run as a boiled/reduced sample
    Lane (c) purified eCG run as a non-boiled/reduced sample
    Lane (d) crude starting material run as a non-boiled/non-reduced
            sample
    Lane (e) purified eCG run as a boiled/non-reduced sample
    Lane (f) purified eCG run as a non-boiled/non-reduced sample

Figure 14

Immunostaining of Western transferred, non-boiled/non-reduced, monoclonal antibody F50-37 affinity purified eCG by:

    (a) F50-37

    (b) F50-75

    (c) F51-99 and

    (d) SBU-4 an unrelated negative control monoclonal antibody that
        recognises a sheep placental antigen (unpublished).

Figure 15

Immunostaining of Western transferred, boiled/non-reduced, monoclonal antibody F50-37 affinity purified eCG by:

(a) F50-37

(b) F50-75

(c) F51-99 and

(d) SBU-4 (negative control monoclonal antibody)

and (e) Amino-black staining of the protein transferred to the nitro-cellulose

P. R. CORFIELD CPA
G. CARTER MA. CANTAB. CPA
R. P. HARDING MA. OXON. CPA
H. L. JUKES B.SC., C CHEM., FRSC.
C PHYS., M.INST.P., CPA

Trade Marks
M. K. PADMORE MITMA

# A. R. DAVIES & Co

EUROPEAN PATENT ATTORNEYS
CHARTERED PATENT AGENTS

BRITISH AND FOREIGN PATENTS DESIGNS
TRADE MARKS AND COPYRIGHT

27 IMPERIAL SQUARE
CHELTENHAM
GL50 1RQ
ENGLAND

TELEPHONE (0242) 57 5 0
TELEX 43 135 PATENT G
FAX GP 2/3 (0242) 570083
CABLES. "PATENTS" CHELTENHAM

ALSO AT OXFORD, LONDON
AND MUNICH

OUR REF.
RPH.P01848EP

YOUR REF

DATE 22nd December 1987

European Patent Office,
Erhardtstrasse 27,
D-8000 Munich 2,
Germany.

EPA EPO-OEB
DG 1
Recu:
12 -01- 1988
09      ANL. ZEICHN.

Dear Sirs,

European Patent Application No. 87309057-5
Bunge (Australia) Pty. Ltd.

ONLY
For the purpose of publication,
correction(s)
allowed
not allowed
Signature:
date: 18. 01. 88
Receiving Section

Furthermore we take this opportunity of requesting correction of a minor error in the specification as follows:

Page 43, line 8 - amend "10,000" to read ">10,000".

Yours faithfully,

R. P. Harding

Encl:

/lcc